# EUROPEAN PATENT SPECIFICATION

(11) **EP 3 274 468 B1**
(45) Date of publication and mention of the grant of the patent: **20.02.2019**
(21) Application number: 16717470.5
(22) Date of filing: 28.03.2016
(51) Int. Cl.: C12Q 1/25

(54) **A METHOD FOR MEASURING THE PROTEASE ACTIVITY OF C3 AND C5 CONVERTASE OF THE ALTERNATIVE COMPLEMENT PATHWAY**
VERFAHREN ZUR BESTIMMUNG DER C3- UND C5- KONVERTASE PROTEASE-AKTIVITÄT IM ALTERNATIVEN KOMPLEMENT-PFAD
PROCÉDÉ DE MESURE DE L'ACTIVITÉ PROTÉASIQUE DES CONVERTASES C3 ET C5 DE LA VOIE ALTERNE DU COMPLÉMENT

(30) Priority: 25.03.2015 US 201562138235 P
(43) Date of publication of application: 31.01.2018
(73) Proprietor: Alexion Pharmaceuticals, Inc., New Haven, CT 06510 (US)
(72) Inventor: JOHNSON, Krista, Southington, Connecticut 06489 (US); FORBES, Christen, North Haven, Connecticut 06437 (US)
(74) Representative: D Young & Co LLP
(86) International application number: PCT/IB2016/051750
(87) International publication number: WO 2016/151557

(56) References cited:
- FISHELSON Z ET AL: "C3 convertase of human complement: Enhanced formation and stability of the enzyme generated with nickel instead of magnesium", JOURNAL OF IMMUNOLOGY 1982 US, vol. 129, no. 6, 1982, pages 2603-2607, XP9190104, ISSN: 0022-1767
- M K PANGBURN ET AL: "The C3 convertase of the alternative pathway of human complement. Enzymic properties of the bimolecular proteinase", BIOCHEMICAL JOURNAL, vol. 235, no. 3, 1 May 1986 (1986-05-01), pages 723-730, XP55273558, GB ISSN: 0264-6021, DOI: 10.1042/bj2350723
- Dana V Devine ET AL: "Regulation of the activity of platelet-bound C3 convertase of the alternative pathway of complement by platelet factor H Communicated by", Proc. Natl. Acad. Sci. USA, 1 January 1987 (1987-01-01), pages 5873-5877, XP055273710, Retrieved from the Internet: URL:http://www.ncbi.nlm.nih.gov/pmc/articl es/PMC298965/pdf/pnas00331-0372.pdf [retrieved on 2016-05-19]
- N. RAWAL ET AL: "C5 Convertase of the Alternative Pathway of Complement. KINETIC ANALYSIS OF THE FREE AND SURFACE-BOUND FORMS OF THE ENZYME", JOURNAL OF BIOLOGICAL CHEMISTRY, vol. 273, no. 27, 3 July 1998 (1998-07-03) , pages 16828-16835, XP055273703, US ISSN: 0021-9258, DOI: 10.1074/jbc.273.27.16828
- BEXBORN ET AL: "The tick-over theory revisited: Formation and regulation of the soluble alternative complement C3 convertase (C3(H2O)Bb)", MOLECULAR IMMUNOLOGY, PERGAMON, GB, vol. 45, no. 8, 21 December 2007 (2007-12-21), pages 2370-2379, XP022513118, ISSN: 0161-5890, DOI: 10.1016/J.JCRYSGRO.2007.07.028

## Description

### TECHNICAL FIELD

This disclosure relates to the fields of immunology, and more specifically to the alternative complement pathway.

### BACKGROUND

The alternative pathway of the complement system plays a role in immunological, inflammatory, coagulation as well as neurodegenerative processes. It is implicated in several human diseases such as age-related macular degeneration, sepsis, cancer, paroxysmal nocturnal hemoglobulinuria ("PNH") and atypical hemolytic uremic syndrome ("aHUS"). A complement-directed drug, a therapeutic C5 antibody (Soliris, Alexion), is the first approved treatment for PNH and aHUS.

The alternative pathway relies on a series of enzymatic steps culminating in cleavage of the complement components C3 into cleavage products C3a and C3b, and C5 into C5a and C5b, by the C3 and C5 convertases respectively. Regulators of the alternative pathway can, among other things, prevent or facilitate formation and activity of the C3 and C5 convertases.

Methods for studying the C3 and C5 convertases, and their regulation often require binding to particles such as zymosan or erythrocytes, and the use of serum as a source of complement proteins. However, serum is poorly defined, and experimental reproducibility may suffer.

There is a need to develop improved methods for studying the complement system *in vitro,* and for identifying regulators of the complement system that may be used to develop new drugs.

### SUMMARY

The embodiements disclosed herein solve the problem discussed above by providing methods for measuring the protease activity of the complement components C5 and C3 convertase, and Factor D in a serum free assay buffer. The disclosure also provides kits to measure the protease activity of C5 and C3 convertase, and Factor D.

One embodiment provides a method for measuring the protease activity of C5 convertase. The method comprises a step of covalently attaching biotin to C3b to produce biotinylated-C3b. The biotinylated-C3b is bound to a biotin binding protein, which is immobilized on a solid phase. The solid phase is incubated in the presence of Factor D, and Factor B in a serum free and gelatin free buffer to form a C5 convertase. C5 is transferred to the buffer with the results that the C5 convertase cleaves C5 to form a C5a and C5b. The amount of C5a can be measured with an immunoassay. The individual components bio-C3b, Factor B, Factor D, and C5 are substantially homogeneous.

Certain embodiments provide a method for measuring the protease activity of C3 convertase. The method comprises a step of covalently attaching biotin to C3b to produce biotinylated-C3b. The biotinylated-C3b is bound to a biotinylated-C3b to a biotin binding protein which is immobilized on a solid phase which is incubating in the presence of Factor D, and Factor B in a serum free and gelatin free buffer to form a C3 convertase. C3 is transferred to the buffer with the results that the C3 convertase cleaves C3 to form a C3a and C3b. The amount of C3a is measured with an immunoassay. The individual components bio-C3b, Factor B, Factor D, and C3 are substantially homogeneous.

Certain other embodiments provide a kit for measuring the protease activity of C5 convertase in a serum free and gelatin free buffer using substantially homogeneous components of the alternative complement pathway. The kit comprises (i) substantially homogeneous biotinylated C3b; (ii) a solid phase coated with a biotin binding protein; (iii) substantially homogeneous Factor B, Factor D and C5; and, (iv) an anti-C5a antibody.

Certain embodiments provide a kit for measuring the protease activity of C3 convertase in a serum free and gelatin free buffer using substantially homogeneous components of the alternative complement pathway. The kit comprises: (i) substantially homogeneous biotinylated C3b; (ii) a solid phase coated with a biotin binding protein; (iii) substantially homogeneous Factor B, Factor D and C3; and, (iv) an anti-C3a antibody.

Numerous other aspects are provided in accordance with these and other aspects of this disclosure. Other features and aspects of the present disclosure will become more fully apparent from the following detailed description and the appended claims.

### DETAILED DESCRIPION

### Brief Description of Figures

**Fig. 1** is a schematic representation of the Alternative Pathway of the complement cascade.
**Fig. 2 (a)** is a schematic illustration of the method of measuring C5 convertase activity on spheres.
**Fig. 2 (b)** is a schematic illustration of the method of measuring C5 convertase activity in a microplate well.
**Fig. 3** is a schematic representation illustrating a method for measuring the Factor D protease activity using Factor B as a substrate.
**Fig. 4** is a schematic illustration of a method for detecting Factor D protease activity for its natural substrate Factor B with a biosensor.
**Fig. 5** is a bar graph, which shows that C5 convertase activity is dependent on the presence of bio-C3b.
**Fig. 6** is a graph, which shows C5 convertase protease activity increased as the amount of bio-C3b was titrated from 0.4 pmoles to 25 pmoles per well of a streptavidin-coated microplate.
**Fig. 7** is a graph, which shows that Factor D activity was inhibited at high isatoic anhydride concentrations.
**Fig. 8** is a recording from a streptavidin coated Bio-Layer Interferometry ("BLI") biosensor showing Factor D cleavage of Factor B and the inhibition of Factor D by 3,4-Dichloroisocoumarin ("DCIC").
**Fig. 9** is a graph showing a dose-response curve of DCIC inhibition of Factor D.
**Fig. 10 (a)** is a graph showing that that the rate of C5 convertase activity on spheres was dependent on the concentration of its substrate, C5.
**Fig. 10 (b)** is a graph showing that that the rate of C5 convertase activity on microplates was dependent on the concentration of its substrate, C5.
**Fig. 11** is a graph showing that as OmCI was titrated from 0 to 200 nM, the C5 convertase activity decreased.
**Fig. 12 (a)** is a graph, which illustrates that the convertase activity on spheres was greater at higher Factor B concentrations.
**Fig. 12 (b)** is a graph, which illustrates that the convertase activity on microplates was greater at higher Factor B concentrations.
**Fig. 13** is a graph, which illustrates that as the concentration of Factor D was increased, the C5 convertase activity increased.
**Fig. 14** is a graph, which illustrates that the C5 activity is dependent on bio-C3b concentration.
**Fig. 15** is a bar graph, which illustrates that the C5 convertase activity decreased as the concentration of bio-C3b was titrated from 0.8x, to 0.1x of the total biotin binding capacity of the streptavidin-coated spheres.
**Fig. 16** is a graph, which illustrates that NiCl₂ is required for C5 convertase activity.

### Definitions

As used herein, the word "a" or "plurality" before a noun represents one or more of the particular noun. For example, the phrase "a mammalian cell" represents "one or more mammalian cells."

The term "homogeneous", or "substantially homogeneous", as applied to a component of the alternative complement pathway means that the component is free or substantially free from contaminating proteins. The extent of homogeneity can be determined by techniques such as gel electrophoresis or other methods well known by those of ordinary skill in the art. A complement component that is greater than 90% homogeneous has less than 10% of contaminating proteins on a pmole by pmole basis. A complement component that is greater than 95% homogeneous has less than 5% of contaminating proteins on a pmole by pmole basis. A complement component that is greater than 99% homogeneous has less than 1% of contaminating proteins on a pmole by pmole basis.

The terms "polypeptide," "peptide," and "protein" are used interchangeably and are known in the art and can mean any peptide-linked chain of amino acids, regardless of length or post-translational modification.

The term "antibody" is known in the art. Briefly, it can refer to a whole antibody comprising two light chain polypeptides and two heavy chain polypeptides. Whole antibodies include different antibody isotypes including IgM, IgG, IgA, IgD, and IgE antibodies. The term "antibody" includes, for example, a polyclonal antibody, a monoclonal antibody, a chimerized or chimeric antibody, a humanized antibody, a primatized antibody, a deimmunized antibody, and a fully human antibody. The antibody can be made in or derived from any of a variety of species, e.g., mammals such as humans, non-human primates (e.g., orangutan, baboons, or chimpanzees), horses, cattle, pigs, sheep, goats, dogs, cats, rabbits, guinea pigs, gerbils, hamsters, rats, and mice. The antibody can be a purified or a recombinant antibody.

The term "antibody" includes "antibody fragment," "antigen-binding fragment," or similar terms are known in the art and can, for example, refer to a fragment of an antibody that retains the ability to bind to a target antigen (e.g., human C5) and inhibit the activity of the target antigen. Such fragments include, e.g., a single chain antibody, a single chain Fv fragment (scFv), an Fd fragment, an Fab fragment, an Fab' fragment, or an F(ab')2 fragment. An scFv fragment is a single polypeptide chain that includes both the heavy and light chain variable regions of the antibody from which the scFv is derived. In addition, intrabodies, minibodies, triabodies, and diabodies are also included in the definition of antibody and are compatible for use in the methods described herein. *See, e.g.,* Todorovska et al. (2001) J Immunol Methods 248(1):47-66; Hudson and Kortt (1999) J Immunol Methods 231(1):177-189; Poljak (1994) Structure 2(12):1121-1123; Rondon and Marasco (1997) Annual Review of Microbiology 51:257-283. An antigen-binding fragment can also include the variable region of a heavy chain polypeptide and the variable region of a light chain polypeptide. An antigen-binding fragment can thus comprise the CDRs of the light chain and heavy chain polypeptide of an antibody. For a detailed discussion on producing human antibodies and human monoclonal antibodies and protocols for producing such antibodies, see PCT publications WO 98/24893; WO 92/01047; WO 96/34096; WO 96/33735; European Patent No. 0 598 877; U.S. Pat. Nos. 5,413,923; 5,625,126; 5,633,425; 5,569,825; 5,661,016; 5,545,806; 5,814,318; 5,885,793; 5,916,771; and 5,939,598.

The term "antibody fragment" also can include, e.g., single domain antibodies such as camelized single domain antibodies. See, e.g., Muyldermans et al. (2001) Trends Biochem Sci 26:230-235; Nuttall et al. (2000) Curr Pharm Biotech 1:253-263; Reichmann et al. (1999) J Immunol Meth 231:25-38; PCT application publication nos. WO 94/04678 and WO 94/25591; and U.S. patent no. 6,005,079. The term "antibody fragment" also includes single domain antibodies comprising two V_{H} domains with modifications such that single domain antibodies are formed.

The term "antibody" also refers to a monoclonal antibodies obtained from a population of substantially homogeneous antibodies. That is, the individual antibodies comprising the population are identical except for possible naturally occurring mutations that may be present in minor amounts. Monoclonal antibodies are highly specific, being directed against a single antigen. Furthermore, in contrast to polyclonal antibody preparations that typically include different antibodies directed against different determinants (epitopes), each monoclonal antibody is directed against a single determinant on the antigen. The modifier "monoclonal" is not to be construed as requiring the production of the antibody by any particular method. For example, the monoclonal antibodies to be used in accordance with the present disclosure may be made by the hybridoma method first described by Kohler et al., Nature, 256: 495 (1975), or may be made by recombinant DNA methods (see, e.g., U.S. Pat. No. 4,816,567). The "monoclonal antibodies" may also be isolated from phage antibody libraries using the techniques described in Clackson et al., Nature, 352: 624-628 (1991) or Marks et al., J. Mol. Biol., 222: 581-597 (1991), for example.

The term "kₐ" is well known in the art and can refer to the rate constant for association of an antibody to an antigen. The term "k_{d}" is also well known in the art and can refer to the rate constant for dissociation of an antibody from the antibody/antigen complex. And the term "K_{D}" is known in the art and can refer to the equilibrium dissociation constant of an antibody-antigen interaction. The equilibrium dissociation constant is deduced from the ratio of the kinetic rate constants, K_{D} = kₐ/k_{d}. Such determinations are typically measured at, for example, 25° C or 37°C. For example, the kinetics of antibody binding to human C5 can be determined at pH 8.0, 7.4, 7.0, 6.5 and 6.0 via surface plasmon resonance (SPR) on a BIAcore 3000 instrument using an anti-Fc capture method to immobilize the antibody.

The term "IC₅₀" is well known in the art and is a measure of the effectiveness of a substance at inhibiting a specific biological or biochemical function. The IC₅₀ is the concentration of the substance at which 50% of the activity of the biological function is inhibited.

The term "serum-free" is well known in the art and refers to a media or buffer prepared without the use of animal serum. The term "gelatin free" refers to media or buffer prepared without gelatin.

The term "biotin-binding protein" or "BBP" refers to proteins that have a high affinity for biotin, such as avidin, streptavidin or neutravidin. The bond between biotin and a BBP, such as streptavidin, is the strongest known non-covalent interaction between a protein and its ligand. Generally, the Kd between a BBP and biotin is from about 10⁻¹⁴ to about 10⁻¹⁵ M.

The term "complex" when used to describe a protein: protein, or a protein:ligand interaction, such as that between biotin and a BBP, refers to a physical state in which the protein:protein or ligand:protein are tightly associated in a binding interaction.

The term "SULFO-TAG™" refers to an amine-reactive, N-hydroxysuccinimide ester which readily couples to the primary amine groups of proteins under mildly basic conditions to form a stable amide bond, and which has the structure:

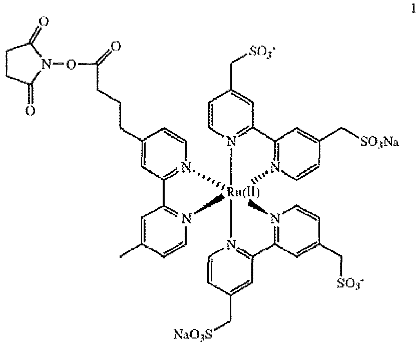

The SULFO-TAG™ reagent is usually used to modify biomolecules for electrochemiluminescence measurement. See US Patent Nos. 7,063,946, and 8,192,926; US Patent Application Nos. 2013/0011860, and 2011/0263451.

The term "Electrochemiluminescence" or "ECL" is a process that uses labels designed to emit light when electrochemically stimulated. Light generation occurs when low voltage is applied to an electrode, triggering a cyclical oxidation and reduction reaction of a heavy metal ion, such as ruthenium. A second reaction component is an electron carrier, such as tripropylamine, which mediates the redox reaction. Because the metal chelate is recycled and the carrier is present in excess, the signal generated from the assay is intensified. The ECL reaction is triggered upon application of an electric potential.

The terms "immobilized" or "bound" encompasses all mechanisms for the binding of antibodies and proteins. Such mechanisms include all mechanisms of receptor-ligand binding, antibody-hapten binding, covalent binding, non-covalent binding, chemical coupling, absorption by hydrophobic/hydrophobic, electrostatic hydrophilic/hydrophilic or ionic interactions and the like.

The term "solid phase" refers to an insoluble material to which one component may be bound or immobilized. The solid phase typically includes, without limitation, any surface commonly used in immunoassays. For example, the solid phase may include the wells of a microplate, spheres or microparticles, made of hydrocarbon polymers such as polystyrene and polypropylene, glass, metals, gels or other materials, the walls of test tubes or membranes.

The term "microplate" as used throughout the specification, refers to a flat plate with multiple "wells" used as small test tubes. Microplates are also known in the art as microtitre plates, microwell plates or multiwell plates. Microplates typically have 6, 24, 96, 384 or 1536 sample wells frequently arranged in a 2:3 rectangular matrix. Some microplates have even been manufactured with 3456 or 9600 wells. Microplates may refer to "array tape" type products that have been developed that provides a continuous strip of microplates embossed on a flexible plastic tape.

Microplates may be manufactured from a variety of materials such as polystyrene, polypropylene, polycarbonate, glass, quartz or, cyclo-olefins. Microplates may be colored for optical absorbance or luminescence detection or black for fluorescent biological assays.

The term "spheres" as used throughout the specification, includes particles that are spherical in shape and generally have a diameter between about 0.01 µm and about 100 µm. The term embraces particles that are referred to in the art as microspheres and nanospheres. Spheres may be manufactured from a wide variety of materials, including ceramics, glass, polymers, and metals. A polymer may be polystyrene. Spheres may be monodisperse.

Unless otherwise defined, all technical and scientific terms used herein have the same meaning as commonly understood by one of ordinary skill in the art to which this disclosure belongs. Methods and materials are described herein for use in the present disclosue; other, suitable methods and materials known in the art can also be used. The materials, methods, and examples are illustrative only and not intended to be limiting.

### The Complement System

The complement system acts in conjunction with other immunological systems of the body to defend against intrusion of cellular and viral pathogens. There are at least 25 complement proteins. Complement components achieve their immune defensive functions by interacting in a series of intricate but precise enzymatic cleavage and membrane binding events. The resulting complement cascade leads to the production of products with opsonic, immunoregulatory, and lytic functions.

The complement cascade can progress via the classical pathway ("CP"), the lectin pathway ("LP"), or the alternative pathway ("AP"). The lectin pathway is typically initiated with binding of mannose-binding lectin ("MBL") to high mannose substrates.

The AP can be antibody independent, and can be initiated by certain molecules on pathogen surfaces. The CP is typically initiated by antibody recognition of, and binding to, an antigenic site on a target cell. These pathways converge at the C3 convertase - the point where complement component C3 is cleaved by an active protease to yield C3a and C3b.

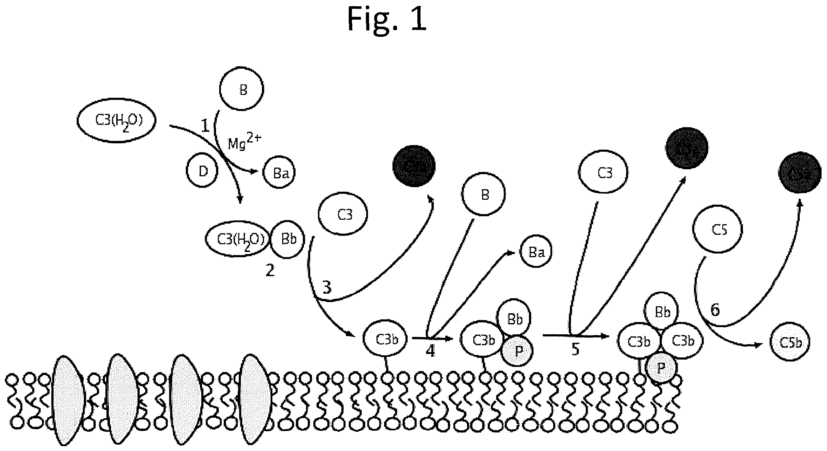

A schematic illustration of the AP is shown in **Fig.** 1.

It is believed that AP C3 convertase is initiated by the spontaneous hydrolysis of complement component C3, which is abundant in the plasma in the blood. This process, also known as "tickover," occurs through the spontaneous cleavage of a thioester bond in C3 to form C3i or C3(H₂O). Tickover is facilitated by the presence of surfaces that support the binding of activated C3 and/or have neutral or positive charge characteristics (e.g., bacterial cell surfaces). This formation of C3(H₂O) allows for the binding of plasma protein Factor B, which in turn allows Factor D to cleave Factor B into Ba and Bb. The Bb fragment remains bound to C3 to form a complex containing C3(H₂O)Bb - the "fluid-phase" or "initiation" C3 convertase. Although only produced in small amounts, the fluid-phase C3 convertase can cleave multiple C3 proteins into C3a and C3b and results in the generation of C3b and its subsequent covalent binding to a surface (e.g., a bacterial surface). Factor B bound to the surface-bound C3b is cleaved by Factor D to thus form the surface-bound AP C3 convertase complex containing C3b,Bb. *See, e.g.,* Müller-Eberhard (1988) Ann Rev Biochem 57:321-347.

The AP C5 convertase is believed to be formed upon addition of a second C3b monomer to the AP C3 convertase. *See, e.g.,* Medicus et al. (1976) J Exp Med 144:1076-1093 and Fearon et al. (1975) J Exp Med 142:856-863. The role of the second C3b molecule is thought to bind C5 and present it for cleavage by the C5 convertase. See, *e*.*g*., Isenman et al. (1980) J Immunol 124:326-331. The AP C3 and C5 convertases are stabilized by the addition of the trimeric protein properdin as described in, e.g., Medicus et al. (1976), *supra.* However, properdin binding is not required to form a functioning alternative pathway C3 or C5 convertase. See, e.g., Schreiber et al. (1978) Proc Natl Acad Sci USA 75: 3948-3952, and Sissons et al. (1980) Proc Natl Acad Sci USA 77: 559-562.

In addition to its role in C3 and C5 convertases, C3b also functions as an opsonin through its interaction with complement receptors present on the surfaces of antigen-presenting cells such as macrophages and dendritic cells. The opsonic function of C3b is generally considered to be one of the most important anti-infective functions of the complement system. Patients with genetic lesions that block C3b function are prone to infection with a broad variety of pathogenic organisms, while patients with lesions later in the complement cascade sequence, i.e., patients with lesions that block C5 functions, are found to be more prone only to *Neisseria* infection, and then only somewhat more prone.

The AP C5 convertase cleaves C5, which is a 190 kDa beta globulin found in normal human serum at approximately 75-100 µg/ml (0.4-0.5µM). C5 is glycosylated, with about 1.5-3 percent of its mass attributed to carbohydrate. Mature C5 is a heterodimer of a 999 amino acid 115 kDa alpha chain that is disulfide linked to a 655 amino acid 75 kDa beta chain. C5 is synthesized as a single chain precursor protein product of a single copy gene (Haviland et al. (1991) J Immunol. 146:362-368). The cDNA sequence of the transcript of this human gene predicts a secreted pro-C5 precursor of 1658 amino acids along with an 18 amino acid leader sequence. See, e.g., U.S. Patent No. 6,355,245.

The pro-C5 precursor is cleaved after amino acids 655 and 659, to yield the beta chain as an amino terminal fragment (amino acid residues +1 to 655 of the above sequence) and the alpha chain as a carboxyl terminal fragment (amino acid residues 660 to 1658 of the above sequence), with four amino acids (amino acid residues 656-659 of the above sequence) deleted between the two.

C5a is cleaved from the alpha chain of C5 by either alternative or classical C5 convertase as an amino terminal fragment comprising the first 74 amino acids of the alpha chain (i.e., amino acid residues 660-733 of the above sequence). Approximately 20 percent of the 11 kDa mass of C5a is attributed to carbohydrate. The cleavage site for convertase action is at, or immediately adjacent to, Arg at amino acid residue 733. A compound that would bind at, or adjacent to, this cleavage site would have the potential to block access of the C5 convertase enzymes to the cleavage site and thereby act as a complement inhibitor. A compound that binds to C5 at a site distal to the cleavage site could also have the potential to block C5 cleavage, for example, by way of steric hindrance-mediated inhibition of the interaction between C5 and the C5 convertase. A compound, in a mechanism of action consistent with that of the tick saliva complement inhibitor, Ornithodoros moubata C inhibitor ('OmCI") (which is a C5 binding protein that can be used in the methods of this disclosure), may also prevent C5 cleavage by reducing flexibility of the C345C domain of the alpha chain of C5, which reduces access of the C5 convertase to the cleavage site of C5. See, e.g., Fredslund et al. (2008) Nat Immunol 9(7):753-760.

C5 can also be activated by means other than C5 convertase activity. Limited trypsin digestion (see, e.g., Minta and Man (1997) J Immunol 119:1597-1602 and Wetsel and Kolb (1982) J Immunol 128:2209-2216) and acid treatment (Yamamoto and Gewurz (1978) J Immunol 120:2008 and Damerau et al. (1989) Molec Immunol 26:1133-1142) can also cleave C5 and produce active C5b.

Cleavage of C5 releases C5a, a potent anaphylatoxin and chemotactic factor, and leads to the formation of the lytic terminal complement complex, C5b-9. C5a and C5b-9 also have pleiotropic cell activating properties, by amplifying the release of downstream inflammatory factors, such as hydrolytic enzymes, reactive oxygen species, arachidonic acid metabolites and various cytokines.

The first step in the formation of the terminal complement complex involves the combination of C5b with C6, followed by C7, and C8 to form the C5b-8 complex at the surface of the target cell. Upon the binding of the C5b-8 complex with several C9 molecules, the membrane attack complex ("MAC", C5b-9, terminal complement complex--"TCC") is formed. When sufficient numbers of MACs insert into target cell membranes the openings create (MAC pores), and mediate rapid osmotic lysis of the target cells. Lower, non-lytic concentrations of MACs can produce other effects. In particular, membrane insertion of small numbers of the C5b-9 complexes into endothelial cells and platelets can cause deleterious cell activation. In some cases activation may precede cell lysis.

C3a and C5a are anaphylatoxins. These activated complement components can trigger mast cell dogranulation, which releases histamine from basophils and mast cells, and other mediators of inflammation, resulting in smooth muscle contraction, increased vascular permeability, leukocyte activation, and other inflammatory phenomena including cellular proliferation resulting in hypercellularity. C5a also functions as a chemotactic peptide that serves to attract pro-inflammatory granulocytes to the site of complement activation.

C5a receptors are found on the surfaces of bronchial and alveolar epithelial cells and bronchial smooth muscle cells. C5a receptors have also been found on eosinophils, mast cells, monocytes, neutrophils, and activated lymphocytes.

While a properly functioning complement system provides a robust defense against infecting microbes, inappropriate regulation or activation of complement has been implicated in the pathogenesis of a variety of disorders, including, e.g., rheumatoid arthritis ("RA"); lupus nephritis; asthma; ischemia-reperfusion injury; atypical hemolytic uremic syndrome ("aHUS"); dense deposit disease ("DDD"); paroxysmal nocturnal hemoglobinuria ("PNH"); macular degeneration (e.g., age-related macular degeneration ("AMD")); hemolysis, elevated liver enzymes, and low platelets ("HELLP") syndrome; thrombotic thrombocytopenic purpura ("TTP"); spontaneous fetal loss; Pauci-immune vasculitis; epidermolysis bullosa; recurrent fetal loss; multiple sclerosis ("MS"); traumatic brain injury; and injury resulting from myocardial infarction, cardiopulmonary bypass and hemodialysis. See, e.g., Holers et al. (2008) Immunological Reviews 223:300-316. Inhibition of complement (e.g., inhibition of terminal complement formation, C5 cleavage, or complement activation) has been demonstrated to be effective in treating several complement-associated disorders both in animal models and in humans. See, e.g., Rother et al. (2007) Nature Biotechnology 25(11):1256-1264; Wang et al. (1996) Proc Natl Acad Sci USA 93:8563-8568; Wang et al. (1995) Proc Natl Acad Sci USA 92:8955-8959; Rinder et al. (1995) J Clin Invest 96:1564-1572; Kroshus et al. (1995) Transplantation 60:1194-1202; Homeister et al. (1993) J Immunol 150:1055-1064; Weisman et al. (1990) Science 249:146-151; Amsterdam et al. (1995) Am J Physiol 268:H448-H457; and Rabinovici et al. (1992) J Immunol 149:1744.

### Methods for Measuring the Protease Activity of Components of the AP

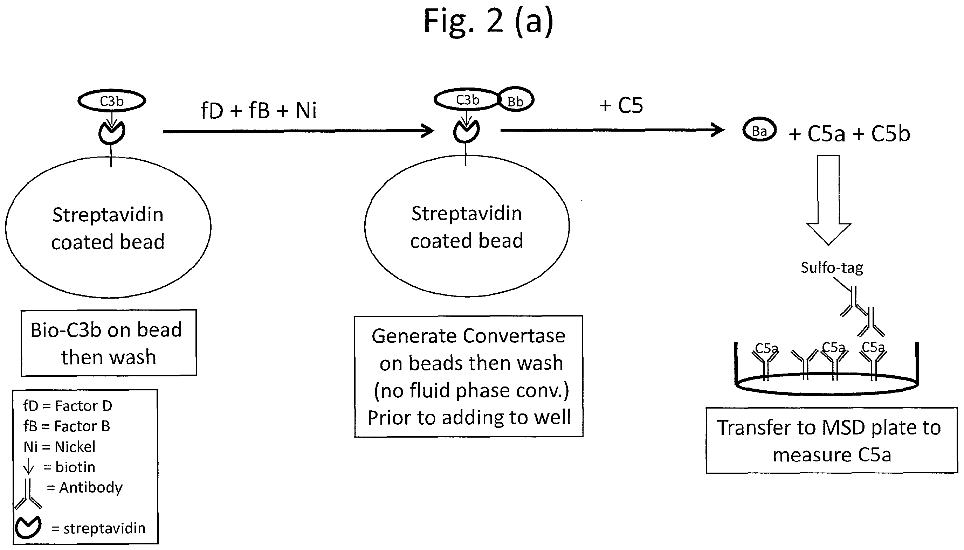

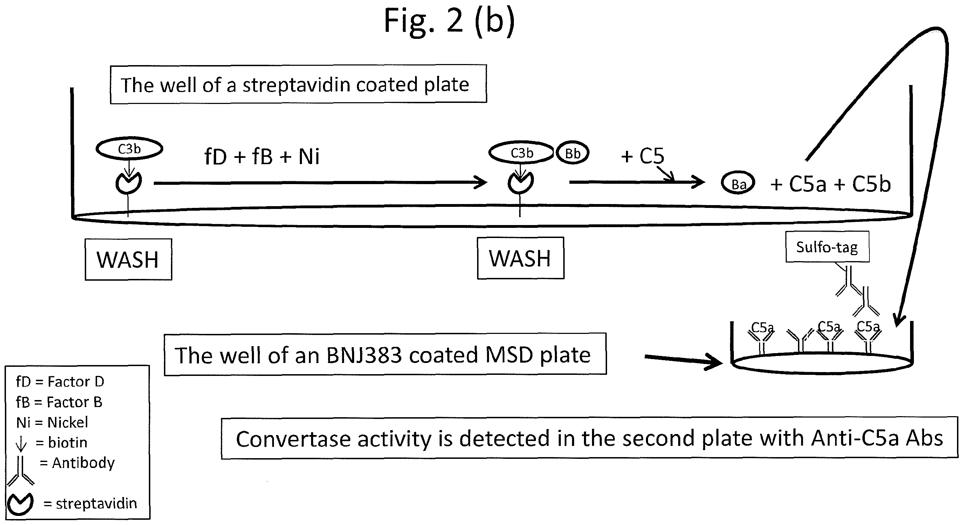

One embodiment provides a method for measuring the protease activity of a convertase immobilized on a solid phase, such as a sphere or a microplate. The convertase may be either a C3 or a C5 convertase. As shown in **Fig. 2 (a)** and **Fig. 2 (b)**, C3b modified with biotin ("bio-C3b") is generally combined with a BBP coated solid phase to form a complex that immobilizes the bio-C3b on a solid phase coated with BBPs. In some embodiments the solid phase may be a sphere and in other embodiments a microplate. In one particular embodiment, the bio-C3b coated solid phase is then incubated with substantially homogeneous Factors D, and B in a serum free and gelatin free buffer to form the convertase. The addition of complement component C3 or C5 results in cleavage by the convertase to form C3a or C5a respectively. The amount of C3a or C5a may be measured with an ELISA or ELISA type assay such as the mesoscale discovery (MSD) ECL assay.

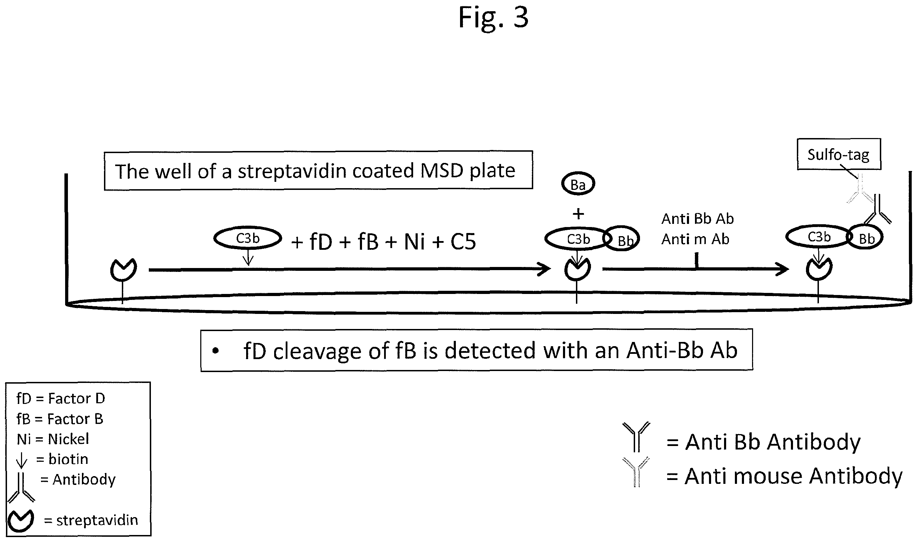

In another embodiment, the present disclosure provides a method for measuring the protease activity of Factor D for its natural substrate Factor B. As shown in **Fig. 3**, the bio-C3b generally binds to a BBP coated solid phase, followed by Factor B binding to the immobilized bio-C3b. Factor D generally cleaves the Factor B into fragments Ba and Bb, and the soluble Ba dissociates from the bio-C3b-Bb complex. The amount of Bb bound to the solid phase may be measured with ELISA, or MSD.

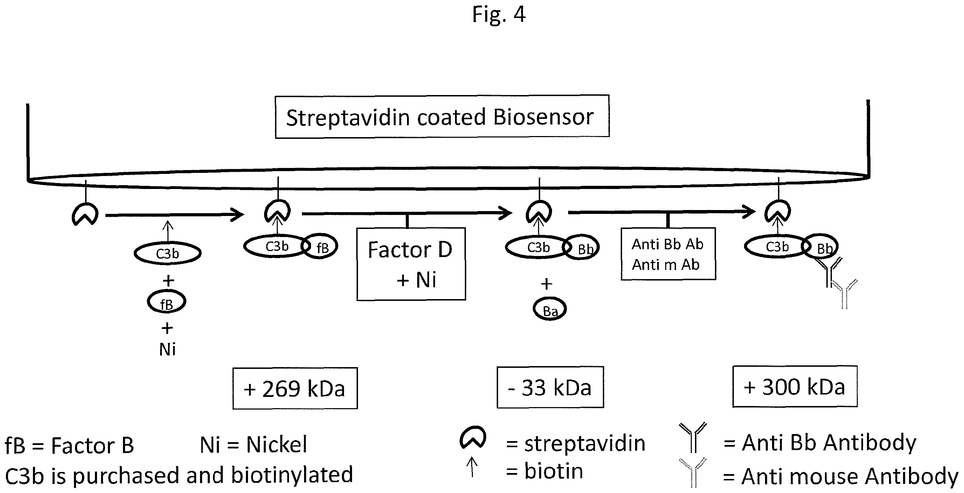

In certain embodiments the present disclosure provides a method of using a biosensor to measure the rate of Factor D proteolysis of Factor B. A particular method is depicted schematically in **Fig. 4****.** Bio-C3b, Factor B and Ni⁺² are generally incubated with a BBP-coated biosensor to form a 269 Kda complex on the surface of the biosensor. As shown in **Fig. 4**, added Factor D cleaves Factor B into its Ba and Bb fragments. Formation of the Bb:bio-C3b complex may be detected with a neo-epitope mouse anti-Bb antibody, which has a high affinity for Bb, but a low affinity for Factor B. Generally, the biosensor detects the binding and dissociation events on its surface, and generates a signal in response to binding. The data generated from the protein binding can be used to calculate kinetic coefficients and IC₅₀ values of inhibitors of Factor D.

### Homogenous Components of the AP

Generally the various embodiments use substantially homogenous AP components including C3b Factor B, Factor D, C3, and C5. The AP components may be isolated from human plasma or they may be recombinantly produced and purified by methods well known in the art. *See* US Patent Nos. 7,858,087 and 6,221,657. Substantially homogenous components of the AP are commercially available from CompTech, QUIDEL™, BIOPUR AG, and SIGMA-ALDRICH®, and others.

Generally the AP components are greater than about 90% homogenous. Frequently, they are greater than about 95% homogenous. The AP components they may be greater than about 99% homogenous.

### Bio-C3b Immobilized on a Solid Phase

In certain embodiment the disclosure provides bio-C3b bound to a BBP immobilized on a solid phase, such as spheres or a microplate.

Biotin is a coenzyme for carboxylase enzymes involved in the synthesis of fatty acids, isoleucine, and valine, and in gluconeogenesis. It has a valeric acid side chain that is frequently used as a point of attachment for the modification of proteins. By derivatizing the acid side chain with various reactive groups, biotin may non-specifically modify amines, sulfhydryls and carboxylic acid side chains of proteins. Spacer arms of polyethylene glycol (PEG) can be used to increase the distance between biotin and the reactive group, which may facilitate the binding of biotin to BBPs.

Proteins can be biotinylated using established protocols well known in the art. See U.S. Patent No. 4,582,810 and the pamphlet entitled Determine reactivity of NHS ester biotinylation and crosslinking reagents, THERMOSCIENTIFIC (2008).

Generally the modification of C3b to form bio-C3b involves incubating a reactive biotin analog with substantially homogenous C3b. Typically the reactive biotin analog is a N-hydroxy succinamide analog of biotin that reacts with primary amines. A particular analog is NHS-(PEG)₄-biotin (ThermoFisher). Adjusting the molar ratio of biotin to C3b may control the extent of labeling. Frequently the biotin to C3b molar ratio may vary from about 10:1 to about 2:1. In one embodiment, the ratio is about 5:1. The reaction is typically performed in an aqueous solution with a non-reactive buffer, such as phosphate buffered saline ("PBS"), or a carbonate-bicarbonate buffer. Optimally the pH ranges from about 6.5 to about 8.5, usually from about 7.0 to about 8.0. Typically the pH is from about 7.2 to about 7.6. The temperature of the reaction may range from about 0° C to about 23° C. The time typically varies from about 20 to about 60 minutes. Bio-C3b is used without purifications and may be stored at -80° C until needed.

The modification of C3b with a biotin analog may be performed with commercially available kits. Representative kits include EZ-LINK (Pierce); LIGHTNING-LINK® (Innova Biosciences Ltd.); and TSA™ Plus Biotin Kits (PerkinElmer).

### Bio-C3b Coated on a Solid Phase

In one embodiment, bio-C3b will be immobilized to a solid phase coated with BBPs. The solid phase may be any surface commonly used in immunoassays, to which a BBP has been coated. The solid phase may include spheres, a microplate wells, or a biosensor.

BBP coating procedures are well known in the art and generally involve either passive adsorption or covalent coupling. See SPHERO™ Technical Note, STN-1 Rev C. 041106, "Particle Coating Procedures"; US Patent Nos. 6,270,983 and 5,061,640. Generally a BBP, such as streptavidin, will adsorb onto polystyrene permanently, or may be covalently coupled to a carrier surface.

Spheres and microplates coated with BBPs are commercially available. Representative coated spheres are sold under the trade name SPHERO™ Nanoparticles (Spherotech), DYNAMICROSPHERES® (Life Technologies), and NANOLINR™ (SOLULINK™). Representative coated microplates are sold under the trade name STREPTAVIDIN GOLD™ (Meso Scale Design), SIGMASCREEN™ (Sigma-Aldrich) and FLASHPLATE® PLUS (PerkinElmer).

During the binding of bio-C3b to BBP, BBP coated spheres are housed in a micro-centrifuge tube, which allows buffer exchange by pelleting the spheres by centrifugation, and resuspending them in a wash buffer.

The binding of bio-C3b to BBP coated spheres generally involves first washing the spheres to remove contaminants from about 2 to about 4 times, with a buffer-detergent solution ("wash buffer") such as 0.1 M PBS, pH 7.4, 0.5% Tween-20 or 10 mM Tris, pH = 8, 0.5% Tween-20.

The spheres are then incubated with bio-C3b. Spheres are at a concentration of about 50 µg/ml to about 1000 µg/ml, or about 500 µg/mL to about 80 µg/ml. In a particular embodiment, the concentration of the spheres is about 200 µg/ml. The bio-C3b is at a concentration of about 30 to about 5000 nM, usually about 300 nM, in 10mM Tris, pH 8.0. Moderate and constant temperatures are normally employed, room temperature is generally sufficient. The incubation time is about 20 to about 30 minutes. After the binding reaction, the spheres are washed about 2 to about 4 times, usually 3 times, with a suitable volume of wash buffer. The pmoles of bio-C3b bound to microspheres will depend on the size of the microspheres.

To coat microplates with bio-C3b the wells are washed about 2 to about 4 times with a suitable volume of a wash buffer. Bio-C3b at a concentration of about 15 nm in 10mM Tris, pH 8.0, may be added directly to each well, and incubated at room temperature for about 20 to about 30 minutes. The wells of the microplate are then washed about 2 to about 4 times, usually 3 times, with a suitable volume of wash buffer.

Typically, a 96-well microplate will have about 0.05 to about 25 pmoles, usually about 0.4 pmoles, of bio-C3b immobilized on the surface of each of well.

Generally, the bio-C3b spheres or plates are used immediately.

### Measuring the Protease Activity of C5 Convertase

A solution, referred to as a "Convertase Solution", comprising Factors D and B, and a divalent cation, is generally added to the bio-C3b coated surface to form the C5 convertase.

Typically, the Convertase Solution includes Factor D at a concentration from about 0.3 to about 20 nM. Alternatively, the concentration of Factor D is about 10 nM.

Generally, the concentration of Factor B is about 1 to about 1500 nM. In one embodiment, Factor B is at a concentration from about 25 to about 500 nM. In another embodiment, the concentration of Factor B is about 400 nM for bio-C3b spheres, and 50 nM for bio-C3b on microplates.

Generally, the divalent cation is Ni⁺² or Mg⁺², usually Ni⁺². In one embodiment, the divalent cation is at a concentration of about 50 to about 5000 µM. In another embodiment, the concentration of divalent cation is about 200 µM.

Optionally, the Convertase Solution may include properdin at a concentration from about 10 nM to about 750 nM, usually about 100 nM to about 500 nM. In one embodiment, the concentration of properdin is about 400 nM.

Optionally, the Convertase Solution may also comprise Factor C3 at a concentration of about 50 nM to about 750 nM. In one embodiment, Factor C3 is at a concentration from about 100 nM to about 500 nM. In another embodiment, it is at about 200 nM.

The C5 convertase may be formed by adding Convertase Solution to the bio-C3b coated spheres or the microplates. The microplate or spheres are incubated at a temperature of about 35° C to about 40° C, usually about 37° C, for about 15 to about 45 minutes with moderate shaking of from about 500 to about 1000 RPMs, usually about 700 RPMs.

Typically, the convertase is formed during the incubation, and the spheres or wells of the microplate are then washed one to two times with a suitable volume of a wash buffer.

Generally, C5 transferred to the Convertase Solution will be cleaved by the convertase to form C5a and C5b. The Convertase Solution with C5 is referred to herein as the "Reaction Solution". This solution is referred to as the "Reaction Solution". Generally, C5 is at a concentration of about 0.1 nM to about 500 nM, in one embodiment about 1 nM to about 250 nM, and in another embodiment about 5 nM to about 100 nM C5. In one embodiment, the Reaction Solution includes a divalent cation such as Mg⁺² or Ni⁺², usually Ni⁺², at about 200 µM.

In one embodiment, the incubation in the Reaction Solution is at a temperature of about 37° C for about 5 to 90 minutes, usually about 30 minutes. During the incubation the sample is subjected to moderate shaking, such as from about 500 to about 1000 RPMs, usually about 700 RPMs.

C5a formation is generally stopped by adding EDTA to the Reaction Solution, binding the divalent cation. The final EDTA concentration will depend on the amount of divalent cation in the reaction mixture, and can readily be determined by those of skill in the art without undue experimentation. Typically, the concentration of EDTA is about 110 mM to about 160 mM EDTA. Alternatively,, the EDTA, will be at a concentration from about 125 mM to about 145 mM. In one embodiment, the concentrations will be about 135 to about 140 mM.

Convertase activity may be assayed by measuring the amount of C5a made by the C5 convertase over a given time period, generally using an immunoassay. Useful immunoassays include ELISA or ELISA-like assays.

ELISA assays, regardless of the detection system employed, generally include the immobilization of an antigen or antibody to a solid phase, as well as the use of an appropriate detecting reagent. Optimal conditions for performing the ELISA can be readily established by those of ordinary skill in the art.

Frequently in an ELISA, an antigen is immobilized to a solid phase and then complexed with an antibody that is linked to an enzyme. Detection may be accomplished by assessing the conjugated enzyme activity via incubation with a substrate to produce a measureable product. ELISAs typically involve chromogenic reporters and substrates that produce some kind of observable color change to indicate the presence of antigen or analyte. ELISA-like techniques use fluorogenic, electrochemiluminescent, and quantitative PCR reporters to create quantifiable signals. These new reporters can have various advantages, including higher sensitivities and multiplexing. In technical terms, newer assays of this type are not strictly ELISAs, as they are not "enzyme-linked", but are instead linked to some nonenzymatic reporter. However, given that the general principles in these assays are largely similar, they are often grouped in the same category as ELISAs.

C5a is a soluble fragment of C5. Methods for measuring C5a with an immunoassay are generally the same whether the C5 convertase is formed on a microplate or with spheres.

In one embodiment, the antibody has a high affinity for the C5a fragment, and a low affinity for the parent C5 component. Optimally, the antibody is neo-epitope antibody. Neo-epitope antibodies generally bind to unique epitopes that are formed on protein fragments as the result of cleavage of a parent protein. For example, a neo-epitope antibody may recognize a newly created N or C terminus of fragments on the C5a fragment but fail to recognize the same sequence of amino acids present in the parent component C5. Generally, the neo-epitope of a cleavage product is not present or is unavailable in the parent protein. In one embodiment, the neo-epitope antibody has a Kd value from about 10⁻⁶ to about 10⁻¹² for the C5a cleavage fragment and a Kd from about 10⁻³ to about 10⁻⁵ for the parent C5 component.

In one embodiment, a neo-epitope specific antibody useful for immobilizing C5a is BNJ383. See PCT publication WO2011/137395).

The detection method for the ELISA may be colorimetric, fluorescent, luminescent, or ECL. Usually the detection method is ECL, and the assay is MSD. Often, the ECL detection method uses a mouse antibody directed to the complement fragment and a goat anti-mouse secondary antibody modified with a SULFO-TAG™ label to detect the amount of C5a immobilized by BNJ383.

Typically, measuring the amount of C5a involves coating the wells of a microplate with a neo-epitope anti-C5a antibody by incubating the anti-C5a antibody in a buffer/detergent solution such as carbonate-bicarbonate buffer at pH 9.4. The microplate is then sealed and incubated at about 37° C for about 30 to about 90 minutes, usually for about 60 minutes. Following incubation, the wells of the microplate are usually washed with a wash buffer to remove non-specifically bound material. The wells of the microplate are then generally blocked with a blocking buffer, such as PBS, 0.5% Tween-20, 0.25% BSA, or PBS/0.05% Tween, 1% Casein, for about 30 to about 90 minutes, usually about 60 minutes. The blocking buffer may also be a commercially available blocking buffer such as Meso Scale Discovery ("MSD") Blocker A, which is a proprietary cocktail of proteins in a PBS-based buffer. Typically, a suitable volume of the Reaction Solution having the C5a fragment and EDTA is transferred to the wells of the microplate coated with BNJ383. The Reaction Solution is incubated for about 15 minutes with shaking at room temperature. Following incubation, the residual Reaction Solution is removed and the wells of the microplate are washed about 1 to 3 times, typically 2 times, with a wash buffer.

In one embodiment, the C5a bound to BNJ383 is measured with a mouse anti-C5a antibody that binds C5a at different sites and a secondary anti-mouse goat antibody modified with SULFO-TAG™, for binding the mouse antibody and generating an ECL signal. Generally, the antibodies are added to the wells of the microplate in 1% MSD Blocking buffer A. The microplate is then incubated in the dark for about 30 minutes after which the plate is washed with PBS, 0.5% Tween-20. A Tris-based buffer containing tripropylamine as a co-reactant for light generation in ECL is added to each well ("Read Buffer"). Frequently the Read Buffer is a commercially available buffer such as MSD Read Buffer T. The microplate ECL signal may be read using a commercial plate reader to determine the amount of C5a. Generally, the amount of C5a may be quantified on a microgram or molar scale by techniques well known to those of skill in the art, such as comparing the ECL signal of the samples to a standard curve.

### Method of Measuring C3 Convertase Activity

Unless, otherwise specified, concentrations, volumes and reaction times and conditions are the same as described above for C5 convertase.

Measuring the activity of C3 convertase is similar to the method of measuring the activity of C5 convertase. Generally, a convertase Solution that includes Factors D and B as well as a divalent cation is added to spheres or microplate wells, with bio-C3b immobilized on the surface. The spheres or microplate are then incubated with moderate shaking to form the C3 convertase after which they are washed one to two times with washing buffer.

Typically, the convertase Solution includes Factor D at a concentration from about 0.3 to about 20 nM. In one embodiment, the concentration of Factor D is about 10 nM.

Generally, the concentration of Factor B is about 1 nM to about 1500 nM. In one embodiment, Factor B is at a concentration from about 25 nM to about 500 nM. In another embodiment, the concentration of Factor B is about 400 nM for bio-C3b spheres, and 50 nM for bio-C3b on microplates.

Generally, the divalent cation is Ni⁺² or Mg⁺², usually Ni⁺². In one embodiment, the divalent cation is at a concentration of about 50 µM to about 5000 µM. In another embodiment, the concentration of divalent cation is about 200 µM.

Optionally, the Convertase Solution may include properdin at a concentration from about 10 nM to about 750 nM, usually at a concentration from about 100 nM to about 500 nM. In a particular embodiment, the concentration of properdin is about 400 nM.

Optionally, the Convertase Solution may also comprise Factor C3 at a concentration of about 50 nM to about 750 nM. In another embodiment, Factor C3 is at a concentration from about 100 nM to about 500 nM. Usually Factor C3 is about 200 nM.

Generally, the spheres or the microplate are then incubated in a Reaction Solution with 10mM Tris, pH 8.0 that includes C3. Usually C3 is at a concentration of about 10 nM to about 750 nM. In one embodiment, the C3 concentration is about 25 nM to about 500 nM. In another embodiment, the C3 concentration is from about 50 nM to about 300 nM. Typically, the solution also has a divalent cation. Generally, incubation is at about 37° C with moderate shaking from about 500 to about 1000, usually about 700 RPMs. The C3 cleavage is generally performed for about 5 to about 90 minutes.

The C3 cleavage reaction is generally stopped by adding EDTA. The final EDTA concentration will depend on the amount of divalent cation in the reaction mixture, and can readily be determined by those of skill in the art.

### Measuring the Amount of C3a with an Immunoassay

The amount of C3a produced is generally measured with an immunoassay using an anti-C3a antibody having a high affinity for C3a, and a low affinity for C3. In one embodiment, the antibody is a neo-epitope anti-C3a antibody. A representative commercially available C3a antibody is provided by HYCULT BIOTECH (clone 2991, Catalog No. HM2074).

Quantifying the amount of C3a produced during the cleavage reaction may be accomplished by coating the wells of a microplate with the anti-C3a antibody. Typically, the Reaction Solution containing the C3a fragment and EDTA is transferred to the coated wells and incubated for about 15 minutes with shaking at room temperature. Following incubation, the residual solution is usually removed and the wells of the microplate are washed about 1 to 3 times, typically 2 times, with wash buffer.

In one embodiment, the detection method is ECL, and the detection reagent is a mouse anti-C3a antibody and a secondary anti-mouse antibody modified with SULFO-TAG™. A Read Buffer comprising tripropylamine is added to the microplate wells and the microplate may be read using a commercial plate reader to measure ECL signal and determine the amount of C3a bound to the anti-C3a antibody.

Generally, the amount of C3a immobilized on the solid surface may be quantified on a milligram or molar scale by comparing the ECL signal of the samples to a standard curve.

### Measuring the Protease Activity of Factor D

**Fig. 3** is a schematic representation illustrating a method for measuring the Factor D protease activity using Factor B as a substrate. Typically, a solution that includes bio-C3b, Factor B, Factor D, and Ni⁺² is added to a microplate well coated with a BBP to form a bio-C3b:Bb complex coating the well. Generally the solution is incubated in the wells of the microplate for about 10 to about 120 minutes, usually about 30 minutes, with gentle mixing.

Generally, the solution comprises about 0.3 to about 60 pmoles of bio-C3b at a concentration of about 30 nM to about 5000 nM, usually about 300 nM. Factor B is typically at a concentration of from about 6 nM to about 1500 nM, usually about 50 nM to about 500 nM. The solution also includes Factor D at a concentration from about 0.3 nM to about 20 nM, usually about 10 nM. The concentration of NiCl₂ is about 50 µM to about 5000 µM. In a specific embodiment, the concentration is about 200 µM of NiCl₂.

Optionally, the Convertase Solution may include properdin at a concentration from about 10 nM to about 750 nM, usually at a concentration from about 100 nM to about 500 nM. In a particular embodiment, the concentration of properdin is about 400 nM.

Optionally, the Convertase Solution may also comprise Factor C3 at a concentration of about 50 nM to about 750 nM. In one embodiment, Factor C3 is at a concentration from about 100 nM to about 500 nM. In another embodiment, Factor C3 concentration is at about 200 nM.

As shown in **Fig. 3**, the bio-C3b binds to the BBP, followed by Factor B binding to the bio-C3b. Factor D cleaves the Factor B into fragments Ba and Bb, and the soluble Ba dissociates from the immobilized bio-C3b-Bb complex. Typically, Ba is removed by washing the wells about 2 to about 4 times, usually 3 times, with a wash buffer.

The amount of Bb produced is may be measured with an ELISA- type MSD assay using an anti-Bb antibody having a high affinity for Bb, and a low affinity for Factor B. In one embodiment, the antibody is a neo-epitope anti-Bb antibody. Anti-Bb antibodies are commercially available. Examples include QUIDEL (A227), ABD SEROTEC (MCA2650), and HYCULT BIOTECH (HM2256). Alternatively, antibodies directed against the Bb fragment of Factor B may be generated using techniques well known to those of skill in the art. See US Patent Application No. 12/675220 and Publication No.2010/0239573, published September 23, 2010.

The detection method for ELISA may be colorimetric, fluorescent, luminescent or by ECL. In a particular embodiment, the detection method is ECL using an antibody modified with SULFO-TAG™, in an MSD format.

As shown in **Fig. 3**, typically a mouse anti-Bb antibody and a secondary goat anti-mouse antibody modified with SULFO-TAG™ are added to each well. The buffer may be 1% MSD Blocking buffer A. Generally, the microplate is sealed and incubated at about 37° C for about 30 to about 90 minutes, usually about 60 minutes. Following incubation, the solution is generally discarded, and the wells of the microplate may be washed with PBS, 0.5% Tween-20. The microplate is usually read using a commercial plate reader to detect the ECL signal. Generally, the amount of Bb immobilized on the surface of each well may be quantified by methods well known to those of skill in the art, such as by generating a standard curve.

### Using a Biosensor to Detect Changes in Factor D Activity

In certain embodiments the present disclosure provides a method of using a biosensor to measure the rate of Factor D proteolysis of Factor B. Generally, the biosensor utilizes bio-layer interferometry (BLI).

Biosensors use label-free technologies to measure biomolecular interactions. Generally, the biosensor detects the binding and dissociation events on its surface, and generates a signal in response to binding. Typically, the biosensor may detect mass addition or depletion, changes in heat capacity, reflectivity, thickness, color or other characteristic indicative of a binding event.

The methods disclosed herein use BLI biosensors. BLI biosensors use an optical analytical technique that analyzes the interference pattern of white light reflected from two surfaces: a layer of immobilized protein on the biosensor tip, and an internal reference layer. Any change in the number of molecules immobilized on the biosensor tip causes a shift in the interference pattern that can be measured in real-time. *See* US Patent No. 7,319,525.

The binding between a ligand immobilized on the biosensor tip surface and an analyte in solution produces an increase in optical thickness at the biosensor tip, which results in a wavelength shift, Δλ (nm), which is a direct measure of the change in thickness of the biological layer. Interactions can be measured in real time, providing the ability to monitor binding specificity, rates of association and dissociation, or concentration.

Only molecules binding to or dissociating from the biosensor can shift the interference pattern and generate a response profile. Unbound molecules, changes in the refractive index of the surrounding medium, or changes in flow rate do not affect the interference pattern.

Generally BLI biosensors coated with streptavidin will bind bio-C3b. Streptavidin coated BLI biosensors fitted to a 96-well format are commercially available under the trade name of DIP AND READ™ (FORTEBIO). See US Patent No. 7,319,525. Streptavidin coated biosensors are commercially available and sold under the trade name of Streptavidin (SA) Biosensors (FORTEBIO, Catalog No. 8-5019).

One embodiment is depicted schematically in **Fig. 4****.** A solution of bio-C3b, Factor B and Ni⁺² are generally incubated with a BBP-coated biosensor forming a 269 Kda complex on the surface of the biosensor.

Optionally, bio-C3b is at a concentration of about 2 µg/ml to about 30 µg/ml per well, usually 10 µg/ml. Factor B may be at a concentration from about 0.1 µM to about 3 µM. In a particular embodiment, Factor D is at a concentration of about 0.6 µM. The solution is usually buffered with PBS containing 0.1% (wt/vol) BSA and 0.02% Tween 20 (vol/vol). (FORTEBIO Kinetics Buffer). The buffer may also contain NiCl₂ at about 0.1 to about 5 mM, often at about 1 mM.

The BBP may be avidin, streptavidin or neutravidin. Usually, the BBP is streptavidin.

Optionally, the solution may include properdin at a concentration from about 10 nM to about 750 nM, usually at a concentration from about 100 nM to about 500 nM. In a specific embodiment, the concentration of properdin is about 400 nM.

Optionally, the solution may also comprise Factor C3 at a concentration of about 50 to about 750 nM. Factor C3 may be at a concentration from about 100 nM to about 500 nM. In one embodiment, it is at about 200 nM.

The biosensors are generally incubated for about 1 to about 10 minutes, usually about 6 minutes. The biosensor records a positive wavelength shift in response to the formation of the Factor B/ bio-C3b complex on the streptavidin.

As shown in **Fig. 4****,** added Factor D cleaves Factor B into its Ba and Bb fragments. In one embodiment, Factor D is at a concentration of from about 0.3 nM to about 20 nM. In another embodiment, the Factor D is at a concentration of about 10 nM.

**Fig. 4** shows the 33 Kda Ba fragment dissociating from the bio-C3b-Bb complex. The biosensor is washed with Kinetics Buffer, over a 2-minute period to remove the soluble Ba fragment. The biosensor will record a negative wavelength shift in response to the loss of the 33 Ba fragment.

Formation of the Bb:bio-C3b complex may be detected with a neo-epitope mouse anti-Bb antibody, which has a high affinity for Bb, but a relatively low affinity for Factor B, and a secondary goat anti-mouse antibody. The antibodies are typically loaded on to the biosensor at a concentration of about 25 nM to about 75 nM, usually 55 nM in Kinetics Buffer. The biosensor is incubated about 1 to about 5 minutes, usually about 2 minutes. The BLI biosensor generally records a positive wavelength shift in response to the binding of the 300 kDa antibody complex.

The data generated from the BLI biosensor-binding assay can be used to calculate kinetic coefficients and IC₅₀ values using commercially available software.

Those of skill in the art will recognize that the AP is a multi-component system that may be studied using the method of the present disclosure, and that the current method will facilitate the discovery of modulators of the pathway. Generally, the component to be examined will be the limiting component in the assay. Thus, the amounts of the other components of the AP should be present at suitable concentration to ensure that the observed reaction is characteristic of and determined by the component to be examined. For example, in a rate determination of a tested component, all other components participating in the reaction must be present at concentrations, which do not limit the reaction rate, so that the concentration of the tested component is "rate-limiting".

### Kits

The present disclosure, provides for kits or sets necessary for measuring the protease activity of complement components, such as C3 convertase, C5 convertase or Factor D, using the methods described herein.

One embodiment of the present disclosure, provides for a kit for measuring the protease activity of C5 convertase. In one embodiment, a kit includes includes:
a. bio-C3b
b. Factor B, and Factor D;
c. a BBP coated solid phase;
d. C5; and,
e. an antibody which binds C5a with a high affinity and, C5 with a low affinity.

An alternative embodiment of the present disclosure provides for a kit for measuring the C3 convertase activity. In one embodiment, a kit includes:
a. bio-C3b
b. Factor B, and Factor D;
c. a BBP coated solid phase;
d. C3, and;
e. an antibody which binds C3a with a high affinity and, C3 with a low affinity.

An alternative embodiment of the present disclosure provides for a kit for measuring the protease activity of Factor D, using the methods described herein. In one embodiment, each kit or set includes
a. bio-C3b
b. Factor B;
c. a BBP coated solid phase;
d. Factor D, and;
e. an antibody which binds Bb with a high affinity, and Factor B with a low affinity.

Another embodiment of the present disclosure provides for a kit for detecting the protease activity of Factor D, using a biosensor. In one embodiment, each kit or set includes
a. bio-C3b,
b. and Factor B;
c. a BBP biosensor;
d. Factor D, and;
e. an antibody which binds Bb with a high affinity, and Factor B with a low affinity.

Each of the kits of the present disclosure may also include properdin.

In one embodiment, the homogeneity of each of the components of the pathway is greater than about 90%. In another embodiment, the homogeneity of each of the components of the pathway is greater than about 95%. In a specific embodiment, the homogeneity of each of the components of the pathway is greater than about 99%.

Typically, the antibody is a monoclonal antibody. Usually the antibody is a neo-epitope antibody having a high affinity for the cleavage fragment protein, and a low affinity for the parent complement component. Optimally, the antibody has a Kd value of from about 10⁻⁶ to about 10⁻¹² for the fragment, and a Kd value of from about 10⁻³ to about 10⁻⁵ for the AP component.

The solid surface may be a well in a microplate. The solid substrate may also be spheres.

The biosensor in some embodiments is a BLI biosensor.

### Examples

For the disclosure to be better understood, the following examples are set forth. These examples are for purposes of illustration only and are not to be construed as limiting the scope of the disclosure in any manner.

Proteins of the AP including Factor B, Factor D, C3, and C5 were purchased from Comptech (Tyler TX).

All microplates used in the Examples were in a 96-well microplate format. Streptavidin coated spheres were purchased from Spherotech (Catalog No. SVP-03-10).

### Example 1

Example 1 exemplifies a method of measuring C5 convertase activity with streptavidin-coated spheres. The results demonstrate that the binding of bio-C3b to streptavidin was a prerequisite for formation of C5 convertase. Results were compared from samples of streptavidin-coated spheres incubated: (i) with bio-C3b; (ii) in the absence of C3b (iii) with non-biotinylated C3b. The method is schematically illustrated in **Fig.** 2 (a).

### Biotinylation of C3b

Bio-C3b was made by reacting C3b with NHS-(PEG)₄-biotin (ThermoFisher), a biotin analog that non-specifically reacts with the primary amine side chain of lysine. The reaction was performed in PBS, pH 7.2 at room temperature for 30 minutes, with a ratio of the NHS-(PEG)₄-biotin to C3b of 5:1. Following biotinylation the bio-C3b was stored at -80° C.

### Bio-C3b Binding to Spheres

Bio-C3b binding to streptavidin on a streptavidin coated spheres produced a robust signal indicative of C5a formation. Streptavidin-coated spheres with C3b (no biotin) or streptavidin-coated spheres were used as two separate controls.

The bio-C3b binding to streptavidin was performed in a 2.0 ml micro-centrifuge tube. Two hundred and forty µg of Spherotech beads, 0.3 mm diameter, streptavidin coated spheres were first washed by centrifugation with 300 µL of 10 mM Tris, pH 8, and then resuspended in 200 µL of 10 mM Tris, pH 8. The spheres were incubated with bio-C3b or C3b at a concentration of 320 nM. Spheres were suspended by gentle shaking at 1350 RPM for 20 minutes at room temperature, and collected by centrifugation at 16 RCF for 10 minutes. The residual supernatant was removed and the beads are then washed to remove residual unbound bio-C3b.

### C5 Convertase

C5 convertase was formed on the spheres by incubating the bio-C3b spheres with a Convertase Solution that included components of the AP, a divalent cation and buffer. The Convertase Solution consisted of Factor D, (10 nM), Factor B (400 nM), and NiCl₂ (200 µM) in 10mM Tris, pH 8.0. The spheres were suspended by gentle shaking at 1350 RPM for 20 minutes at 37°C. The spheres were centrifuged at 16 x g (RCF) for 10 minutes, the supernatant is removed, and the spheres are resuspended in 1.2 ml of 10 mM Tris pH 8, 1 mM EDTA to a final concentration of 200 µg/ml.

### Cleaving C5 into C5a and C5b

Formation of C5a was accomplished by incubating C5, and the spheres in a Reaction Solution that included a divalent cation. The Reaction Solution was prepared by transferring to a microplate well: (i) 2 µg of spheres with C5 convertase in 10 µl of the Convertase Solution; (ii) 20 µL of a 500 µM NiCl₂ solution; and (iii) 20 µL of a 250 nM solution of C5 (5 pmoles). The microplate was sealed and incubated at 37°C for 30 minutes with gentle shaking at 700 RPM. Following the incubation, the reaction was stopped by adding 10 µL of a 500 mM EDTA in 10 mM Tris, pH = 8.0. (Total Volume = 60 µl)

### Measuring C5a With An ELISA

The amount of C5a in the Reaction Solution was measured with an MSD assay, similar to an ELISA. The neo-epitope anti-C5a antibody, BNJ383, captured soluble C5a, binding it to a well in a microplate. BNJN383 has a high affinity for C5a but a low affinity for C5. The detection reagent was a mouse anti-C5a antibody that binds the C5a captured on the plate, and a SULFO-TAG™ modified anti-mouse secondary antibody that bound the mouse antibody, and generated an ECL signal.

To wells of a high binding 96-well microplate (MSD Catalog No. L15XB-3) was added BNJ383 (0.3 pmoles,)2 µg/mL in 25 µL of a carbonate-bicarbonate buffer at pH 9.4. The microplate was sealed and incubated at 37° C for 60 minutes. Following incubation, the residual solution was removed, and the wells were washed three times with 300 µL of PBS, 0.5% Tween-20. The wells were blocked with 150 µL of 1% MSD blocker A, in PBS, for 60 minutes at room temperature. The residual solution was then removed, and the wells were washed 3 times with 300 µl of PBS, 0.5% Tween-20. The Reaction Solution (60 µl) with the soluble C5a fragment was added to the wells, which were incubated for 15 minutes with gentle shaking (500 rpm) at room temperature.

The residual Reaction Solution was removed and the wells were washed 3 times with 300 µl of PBS, 0.5% Tween-20. To the washed wells was added 2 µg/mL of anti-C5a antibody (HYCULT BIOTECH 2942, catalog No. HM2078) and 0.5 µg/mL SULFO-TAG™ modified goat-anti mouse antibody (MSD, Catalog No. R32AC-5) in 25 µl of 1% MSD Blocker Solution A.

The wells were sealed with a foil seal and incubated in the dark for 30 minutes. Following incubation, the wells were washed 3 times with 300 µL of PBS, 0.5% Tween-20. One hundred and fifty µL of 2X MSD Read Buffer containing tripropylamine as an electron carrier was added to the wells using a negative pipetting technique. The microplate was read on an MSD imager to measure the ECL signal generated by the SULFO-TAG™ affixed to the goat secondary antibody.

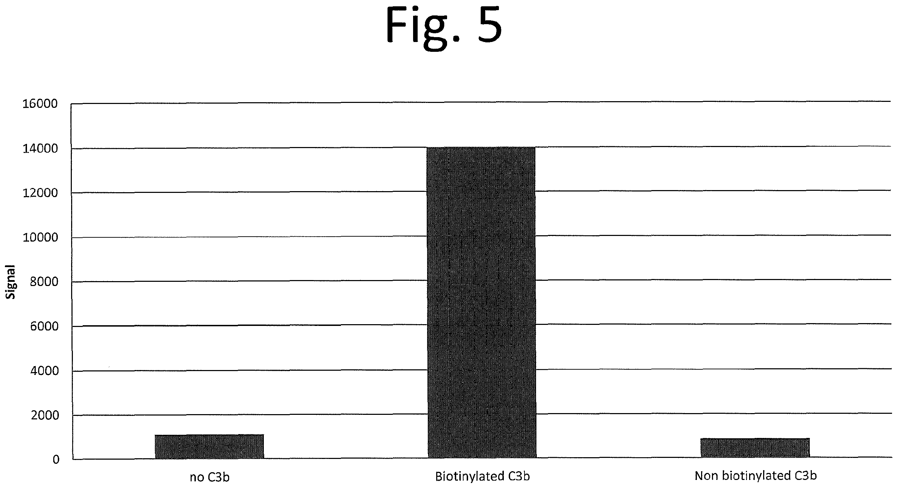

**Fig. 5** is a bar graph of the results, which shows that C5 convertase activity was dependent on the attachment of bio-C3b to the streptavidin-coated spheres. The Y-axis shows the ECL signal, which reports the formation of C5a resulting from the cleavage of the C5 component by C5 convertase. The X-axis shows the tested conditions for each sample. Streptavidin coated spheres incubated with bio-C3b had an ECL signal of about 14,000. In contrast samples incubated in the absence of C3b, or in the presence of non-biotinylated C3b had an ECL signal of about 1000. The ECL signal of 14,000 reported the formation of the C5a fragment by C5 convertase, whereas the ECL of about 1000 indicated the absence of C5a, and that C5 convertase had not formed.

### Example 2

### C5 Convertase on Streptavidin Coated Microplates

Example 2 exemplifies the method of preparing C5 convertase on microplates.

In this Example, individual streptavidin coated wells were incubated with different amounts bio-C3b, ranging from 0.4 pmoles to 25 pmoles. The results showed that the amount of C5a produced was dependent on the amount of bio-C3b immobilized in the wells. The method is schematically illustrated in **Fig.** 2 (b).

### Bio-C3b Binding to Microplate Wells

Bio-C3b binding to streptavidin on a streptavidin-coated microplate produced a bio-C3b coated microplate.

The wells of a streptavidin coated microplate were washed 3 times with 300 µL of PBS, 0.5% Tween-20. The following amounts of bio-C3b were added to individual wells in 25 µl of 10mM Tris, pH 8.0: 0.4 pmoles; 0.8 pmoles; 1.6 pmoles; 3.1 pmoles; 6.3 pmole; 12.5 pmoles; or 25 pmoles. The microplate was sealed and incubated with gentle shaking at room temperature for 30 minutes. The wells were then washed twice with 200 µl of PBS, 0.5% Tween-20.

### C5 Convertase

C5 convertase was formed in microplate wells by incubating bio-C3b coated wells with a Convertase Solution that included components of the AP, a divalent cation and buffer. The Convertase Solution was prepared with Factor D (10 nM), Factor B (50 nM), and NiCl₂ (200 µM) in 10 mM Tris, pH 8.0. Fifty µL of the Convertase Solution was added to the washed wells, and the microplate was sealed and shaken at 700 RPM at 37° C for 15 minutes. The wells were washed twice with 200 µl of PBS, 0.5% Tween-20.

### Cleaving C5 to C5a and C5b

Formation of C5a was accomplished by incubating C5 in the wells with a Reaction Solution that included a divalent cation. The Reaction Solution was prepared by transferring 20 µl of a 100 nM C5 solution in 10 mM Tris, pH 8.0, to the wells and adding 20 µl NiCl₂. The microplate was sealed and incubated at 37° C with shaking at 700 RPM. The reaction was stopped with 15 µl of an EDTA solution at pH 8.0. The final EDTA concentration was 137 mM. (Total Volume = 55 µl.)

### Measuring C5a with MSD (an ELISA-type assay)

The amount of C5a in the Reaction Solution was measured with an MSD assay. The neo-epitope anti-C5a antibody, BNJ383, affixed to a microplate well, captured soluble C5a,. The detection reagent was a mouse anti-C5a antibody that binds the C5a captured on the plate, and a SULFO-TAG™ modified anti-mouse secondary antibody that bound the mouse antibody, and generated an ECL signal.

The wells of a 96-well microplate were coated with BNJ383 by incubating 25 µL of a solution with the antibody at a concentration of 2 µg/ml in carbonate-bicarbonate coating buffer at pH = 9.4. The microplate was sealed and incubated at 37° C for 60 minutes. Following incubation, the residual antibody solution was discarded, and the wells of the microplate were washed 3 times with 300 µL of PBS, 0.5% Tween-20. The wells of the microplate were then blocked with 150 µL of 1% MSD Blocker A, in PBS for 60 minutes at room temperature. The residual blocking solution was removed and the wells of the microplates were washed 3 times with 300 µL of PBS, 0.5% Tween-20. Fifty-five µL of the Reaction Solution was added to each blocked well, and incubated 15 minutes with shaking at room temperature. The residual Reaction Solution was removed and the wells were washed 3 times with 200 µL of PBS, 0.5% Tween-20. Twenty-Five µl of the detection reagent was added to each well (2 µg/mL anti-C5 Ab 2942 (HYCULT BIOTECH) and 0.5 µg/mL SULFO-TAG™ modified goat-anti mouse antibody (MSD). The wells were sealed with a foil seal and incubated in the dark for 30 minutes. Following incubation, the wells were washed 3 times with 300 µl of PBS, 0.5% Tween-20, followed by the addition of 150 µl of Read Buffer. The ECL signal was measured on an MSD imager to determine the amount of C5a produced.

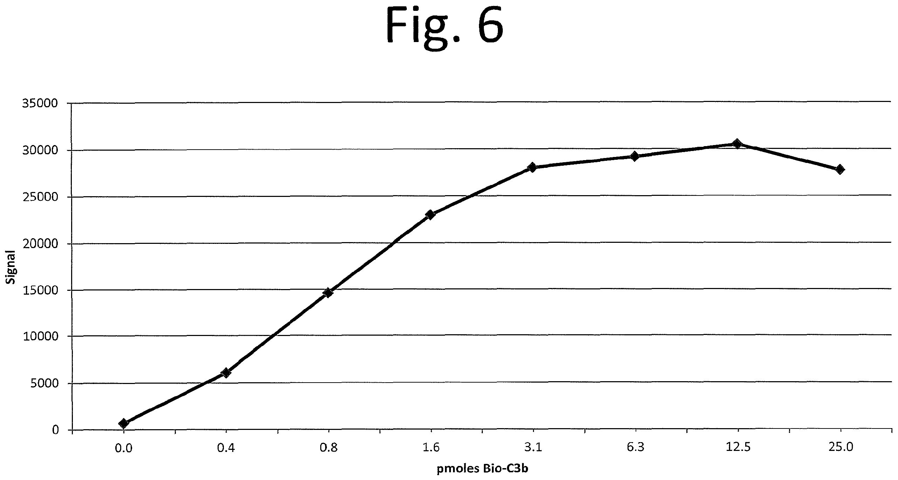

**Fig.** 6 is a graph, which demonstrates that the amount of C5a produced was a function of the concentration (pmoles) of bio-C3b immobilized in the microplate wells. The Y-axis shows the ECL signal from the secondary antibody reporting the amount of C5a. The X-axis shows the amount of bio-C3b in pmoles. The ECL signal increased as the amount of immobilized bio-C3b increased from 0.4 pmoles to 25 pmoles. Samples incubated with 0.4 pmoles of bio-C3b had an ECL signal of about 6000. At 12.5 pmoles, the ECL signal plateaued at about 30,000. Samples incubated in the absence of bio-C3b had a relative signal of about 1,000.

### Example 3:

Example 3 exemplifies the method of measuring Factor D activity with a BBP coated plate. This process includes the steps of: (i) binding Factor B to bio-C3b coated plates; (ii) Factor D cleavage of Factor B into fragments Ba and Bb; and, (iii) measuring the Bb product with an MSD assay. The method is schematically illustrated in **Fig. 3****.**

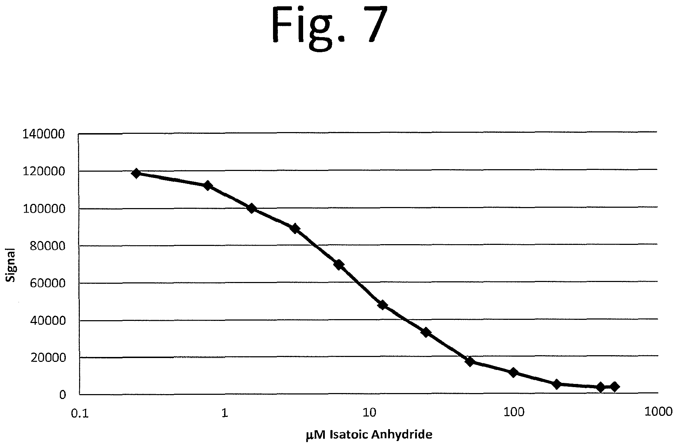

Also exemplified is the effect of the Factor D inhibitor isatoic anhydride on the production of the Bb fragment. The results shown in **Fig. 7****,** demonstrate that the activity of Factor D decreased with increasing concentrations of isatoic anhydride.

### Forming Bb On Bio-C3b Coated Plates

An initial blocking step was performed to reduce non-specific binding. One hundred and fifty µl of 1% MSD blocking buffer A was added to the wells of a streptavidin-coated microplate. The microplate was incubated for 1 hour at room temperature. The wells were then washed three times with 300 µl PBS, 0.5% Tween-20.

Forming Bb immobilized on a bio-C3b coated plate was accomplished by incubating bio-C3b (28nM) Factor R (75nM), Factor D (0.8nM), 1mM NiCl₂ in 50 µl of 10mM Tris, pH 8.0. Samples were incubated in the presence of isatoic anhydride at concentrations ranging from 1 µM to 1000 µM. The microplate was sealed and incubated at room temperature for 30 minutes. The wells were washed one time with PBS, 0.5% Tween-20.

### Measuring Bb with an MSD assay

The amount of Bb fragment binding to the surface of the microplate was measured with an ELISA using a mouse anti-Bb antibody and a secondary goat anti-mouse antibody modified with SULFO-TAG™ modified to generate an ECL signal.

Twenty-five microliters of an antibody solution was added to the washed wells, comprising 2 µg/mL of the anti-Bb antibody (Quidel A227) and 0.5 µg/mL of SULFO-TAG™ modified goat-anti mouse secondary antibody (MSD, R32AC-5) in 1% MSD blocking buffer A. The microplate was sealed with foil and incubated in the dark for 30 minutes. Following incubation, the wells were washed 3 times with 300 µL PBS, 0.5% Tween-20, and 150 µL of Read Buffer was added to each well using a negative pipetting technique. The ECL signal was measured on an MSD imager to determine the amount of Bb produced by Factor D.

**Fig. 7** is a graph of the results of Example 3. The Y-axis shows the ECL signal generated by the secondary antibody. The X-axis shows the concentration of isatoic anhydride. The results show concentration dependent isatoic anhydride inhibition of Factor D.

The ECL signal reports the amount of Bb fragment produced by Factor D proteolysis. The signal decreased from about 120,000 to about 2000 as the isatoic anhydride was titrated from 0.3 µM to 1000 µM. At 0.3 µM isatoic anhydride, Factor D was fully active and cleaved Factor B into its Ba and Bb fragments, which resulted in the generation of an ECL signal of 120,000. At 1000 µM isatoic anhydride, Factor D was essentially completely inhibited, and unable to cleave Factor B. The ECL signal was less than about 2000.

### Example 4

Example 4 exemplifies the use of a BLI biosensor to detect changes in Factor D activity. This Example also shows the inhibitory effect of DCIC on Factor D, and the formation of the Bb fragment. The method is schematically illustrated in **Fig. 4****.**

All measurements were recorded on an OCTET® RED System set to the advanced quantitation mode. The biosensors were SA-Streptavidin DIP AND READ™ configured to a 96-well microplate format (FORTEBIO). All loading volumes were 200 µl.

As shown in **Fig. 4****,** the first step was to form the bio-C3b/ Factor B complex on the surface of the biosensor. Eight streptavidin-coated biosensors were incubated with bio-C3b (10 µg/ml), and Factor B (0.6 µM) in Kinetics Buffer with 1 mM NiCl₂ for 6 minutes.

To form bio-C3b/Bb, each biosensor was loaded with a mixture of BioC3b (10 µg/mL), factor B (600 nM) and Ni²⁺ (1 mM) in Kinetics Buffer. Each biosensor was then incubated with factor D (30 nM)mixed with DCIC at one of 8 different concentrations, ranging from 25 µM to 400 µM. The incubation time was 2 minutes.

The presence of Bb on the biosensor was detected by loading the biosensors with 55 nM of an anti-Bb antibody (Quidel, A227) and 55 nM Goat anti-mouse secondary antibody in Kinetics Buffer. The incubation time was 2 minutes.

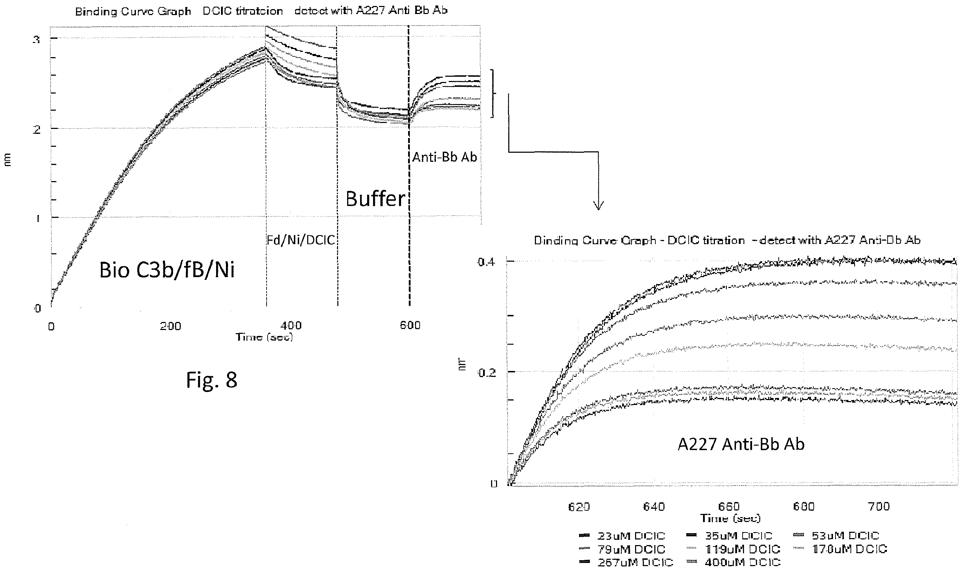

A recording from the biosensor is shown in **Fig. 8****.** The binding and dissociation of proteins from the biosensor resulted in a wavelength shift. The Y-axis shows the wavelength shift, Δλ (nm). The X-axis is time measured in seconds.

From time 0 to about 360 seconds (∼6 minutes), the 269 kDa bio-C3b/ Factor B complex formed on the streptavidin coated biosensor. This was recorded on the biosensor as a positive wavelength shift of about 2.9 nM. From about 360 to about 480 seconds (∼2 minutes), Factor D cleaved Factor B into fragments Ba and Bb. The dissociation of the 33 kDa Ba fragment was recorded as a negative wavelength shift of about 0.2 nM. The biosensors were washed to remove residual Ba and DCIC from about 480 to 600 seconds (∼2 minutes), resulting in a further negative wavelength shift. The binding of the 300 kDa primary and secondary antibody complex to the Bb fragment was recorded as a positive wavelength shift from about 600 to 720 seconds (∼2 minutes). The positive wavelength shift ranged from about 0.1 nM to about 0.4 nm.

The highest concentration of DCIC, 178 µM, 267 µM, and 400 µM, are shown in **Fig. 8** as the purple, light blue and orange traces respectively. At these concentrations, DCIC maximally inhibited Factor D. A relatively modest wavelength shift of about 0.151 nm reflects the relatively small amount of antibody bound to the Bb fragment. In contrast, at 23 µM and 35 µM, DCIC had a minimal effect on Factor D activity, and there was relatively large amount of antibody binding, resulting in a positive wavelength shift of about 0.4 nm. At concentrations of DCIC between 400 µM and 23 µM, the biosensor recorded a positive wavelength shift between 0.15 and 0.4 nm.

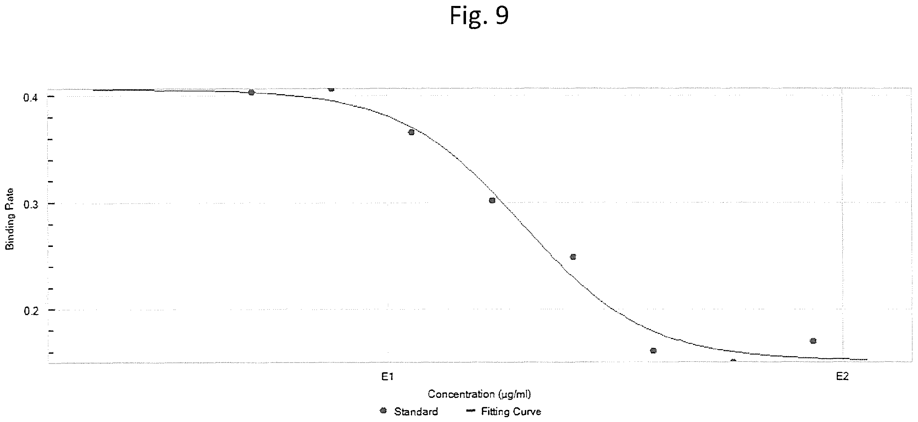

**Fig. 9** shows the effect of DCIC on the rate of antibody binding calculated from the data shown in **Fig. 8****.** The Y-axis represents the binding rate and the X-axis is the concentration of DCIC. The data was used to calculate the DCIC IC₅₀ on the rate of antibody binding. The IC₅₀ for DCIC was 19.8 µg/ml.

### Example 5

Example 5 demonstrates that the rate of C5 convertase activity was dependent on the concentration of its substrate, C5. Results are shown for both streptavidin coated spheres and streptavidin coated microplates.

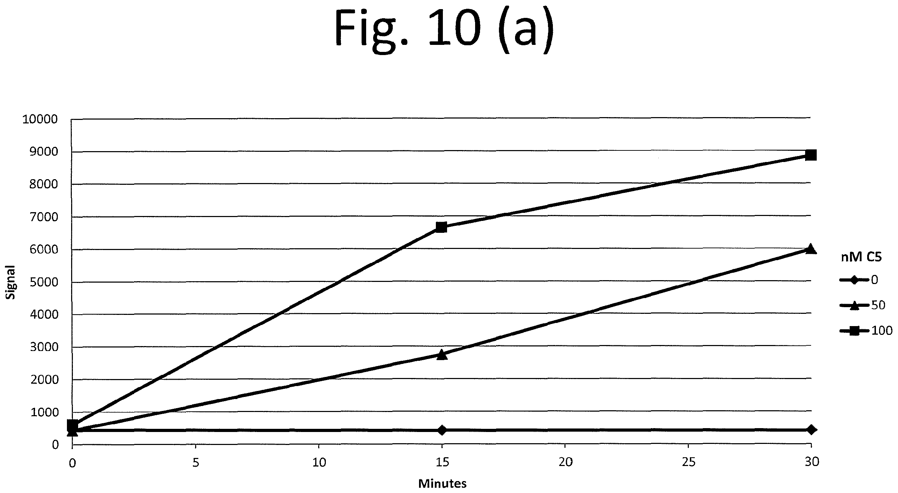

**Fig. 10 (a)** is a graph showing that on spheres the rate of C5 convertase activity was dependent on the concentration of C5. The X-axis shows the time in minutes and the Y-axis shows the ECL signal from the SULFO-TAG™ secondary antibody. The ECL signal reports the amount of C5a produced by C5 convertase.

The concentration of C5 was 0, 50 and 100 nM. Spheres with C5 convertase were prepared as in Example 1. The concentrations of AP components were: bio-C3 spheres (200 µg/ml); Factor B (400 nM); C5 (0, 50, and 100 nM); Factor D (10 nM); and NiCl₂ (200 µM).

As the concentration of C5 increased, the ECL signal increased. Results for up to 30 minutes are shown.

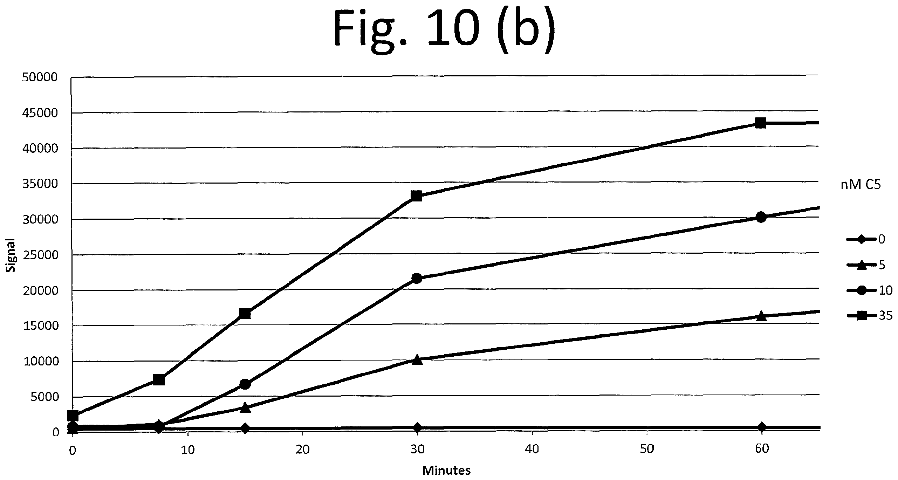

**Fig. 10 (b)** is a similar graph showing the effect of C5 concentration on C5 convertase activity, when bound to a microplate. The X-axis shows the time in minutes and the Y-axis shows the ECL signal.

Microplates having C5 convertase immobilized on the surface were prepared as in Example 2. The concentration of AP components was: Factor B (50 nM), C5 (0, 5, 10, and 35 nM), Factor D (10 nM), and NiCl₂ (200 µM).

As the concentration of C5 was increased, the ECL signal increased. Results up to 60 minutes are shown.

### Example 6

Example 6 demonstrates that OmCI, a C5 binding protein, inhibits C5 convertase activity on spheres.

Spheres having C5 convertase immobilized on the surface were prepared as in Example 1. Bio-C3b spheres (200 µg/ml); Factor B (50 nM); C5 (100 nM), and Factor D (10 nM) and NiCl₂ (200µM) were incubated for 60 minutes in the presence of OmCI at 0, 25, 50, 100, 150 and 200 nM.

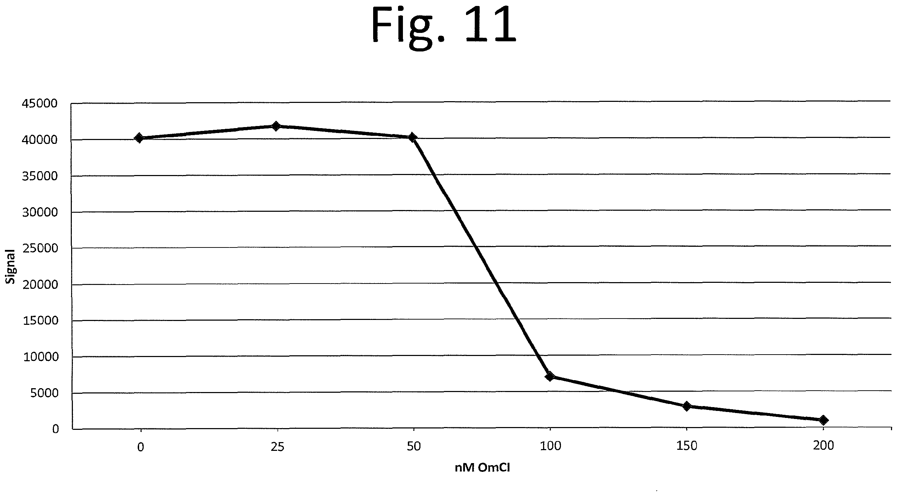

**Fig. 11** is a graph showing the inhibitory effect of OmCI on C5 convertase activity. The Y-axis shows the ECL signal from the SULFO-TAG™ labeled goat secondary antibody. The X-axis shows the concentration of OmCI. The ECL signal reports the amount of C5a produced by C5 convertase. The graph shows that as OmCI was increased from 0 to 200 nM, the amount of C5a produced by the convertase decreased.

OmCI is a C5 binding protein that reduces access of the C5 convertase to the C5 cleavage site. The graph shows that at a concentration of 50 nM, OmCI had essentially no effect on convertase activity. The ECL signal of 40,000 was nearly identical to the signal obtained when the OmCI concentration was 0, indicating that nearly as much C5a was being produced in the presence of 50 nM OmCI, as in the absence of OmCI.

However at a concentration of 100 nM, the ECL signal dropped 8-fold to 5000, demonstrating that OmCI decreases C5a production.

### Example 7

Example 7 demonstrated that the C5 convertase activity was dependent on the concentration of Factor B. Results are presented for both spheres and microplates.

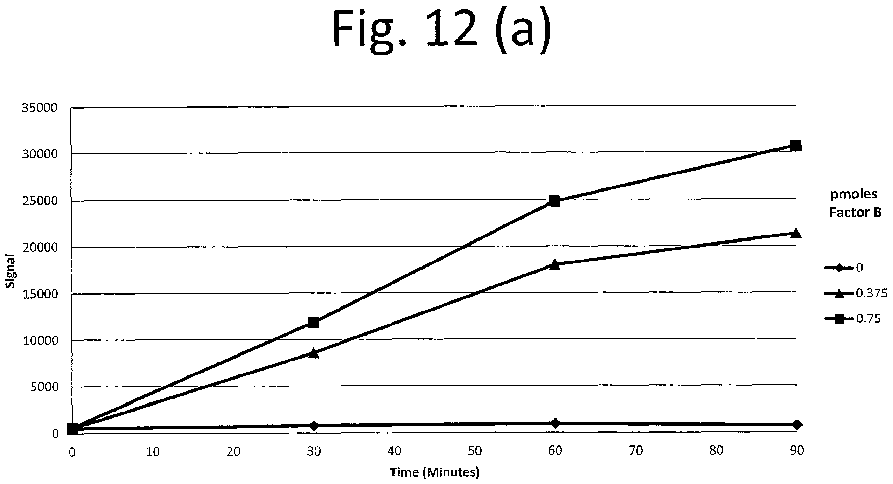

**Fig. 12 (a)** is a graph, showing the effect of Factor B on C5 convertase activity with spheres. The Y-axis shows the ECL signal from the secondary antibody and, the X-axis shows the time in minutes. The ECL signal reports the amount of C5a produced by C5 convertase. Spheres with bound C5 convertase were prepared as in Example 1. Spheres were at a concentration of 2 µM, Factor B (0, 0.375 or 0.75 pmoles), C5 (100 nM), Factor D (10 nM), and NiCl₂ was 200 µM. The graph shows that the ECL signal was greater at 0.75 pmoles of factor B than at 0.375 pmoles, demonstrating that increasing Factor B increased C5 convertase activity. Results are shown at 30, 60, and 90 minutes.

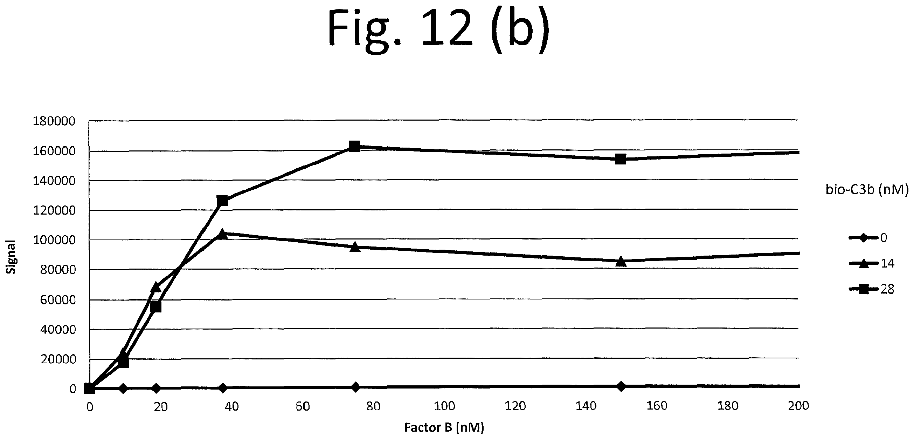

**Fig. 12 (b)** is a graph showing the effect of Factor B on C5 convertase activity using microplates. The Y-axis shows the ECL signal from the secondary antibody, and the X-axis shows the concentration of Factor B. Microplates having C5 convertase immobilized on the surface were prepared as in Example 2. Results are shown with bio-C3 (0, 14 and 28 nM), Factor B (0, 10, 20, 37.5, 75, and 150 nM), C5 (100 nM), and Factor D (10 nM). The time of the reaction was 20 minutes.

The graph shows that as the concentration of Factor B was increased from 0 to 150 nM, the ECL signal reporting C5a formation increased. It also shows that C5a formation was dependent on bio-C3b. In the absence of bio-C3b there was no convertase activity; the activity was greater at 28 nM, than at 14 nM bio-C3b.

### Example 8

Example 8 demonstrates that the C5 convertase activity is dependent on the concentration of Factor D.

Spheres with bound C5 convertase were prepared as in Example 1. Results are shown for bio-C3b spheres (200 µg/ml), Factor B (400 nM) C5 (100 nM), Factor D (0, 0.625 or 20 nM), and NiCl₂ (200 µM).

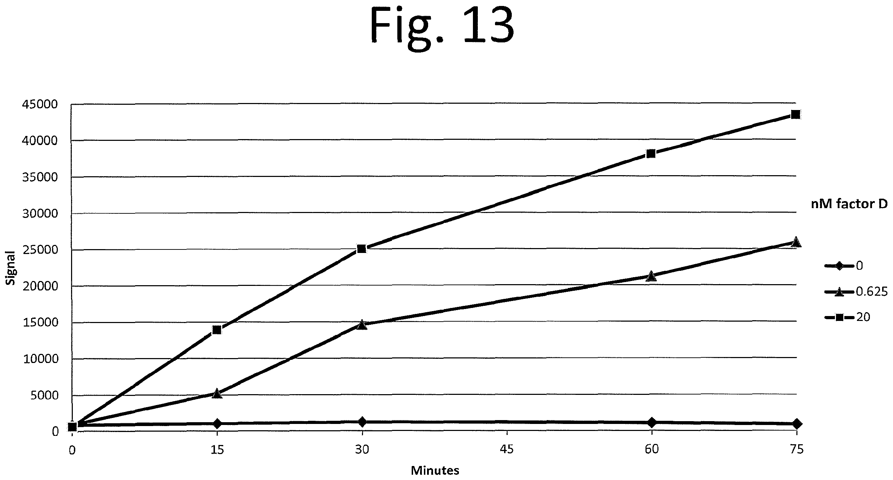

**Fig. 13** is a graph, which illustrates that as the concentration of Factor D was increased, the C5 convertase activity increased. The Y-axis shows the ECL signal from the secondary antibody. The X-axis shows time in minutes.

The ECL signal reports the amount of C5a produced by C5 convertase. The signal is greater for samples with Factor D at 20 nM than at 0.625 nM.

### Example 9

Example 9 demonstrates that the C5 convertase activity is dependent on the concentration of the bio-C3b-streptavidin-coated spheres ("bio-C3 spheres").

Spheres with bound C5 convertase were prepared as in Example 1.

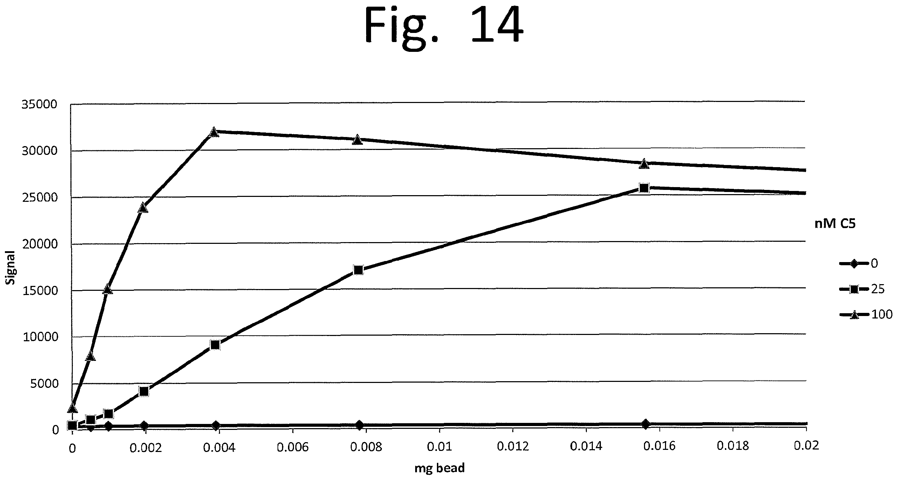

**Fig. 14** is a graph, which illustrates that the C5 activity is dependent on bio-C3b. The Y-axis shows the ECL signal from the SULFO-TAG™ labeled goat secondary antibody. The X-axis shows the mg of bio-C3b spheres incubated in the reaction.

As bio-C3b spheres were titrated from 0 to 16 µg, the C5 convertase activity increased in the presence of C5. These data demonstrate the C5 convertase activity is dependent on the amount of bio-C3b spheres in a reaction.

### Example 10

Example 10 demonstrates that the C5 convertase activity is dependent on the concentration of bio-C3b on spheres.

Spheres with bound C5 convertase were prepared as in Example 1.

Streptavidin-coated spheres (0.5 ml/ sample, 200 µg/ml) were incubated with varying concentrations of bio-C3b. The concentration of bio-C3b in the reaction was 260 pmoles/ml for the 0.8x; 130 pmoles/ml for 0.4x; 65 pmoles/ml for 0.2x; and 32.5 pmoles/ml for 0.1x, where x is the total biotin binding capacity of the streptavidin-coated spheres (X is 1620 pmoles per milligram). C5a was generated by incubating spheres with varying amounts of bio-C3b, Factor B (50 nM), C5 (200 nM), Factor D (10 nM), and NiCl₂ (200 µM). The reaction time was 60 minutes.

C5a detection was with an MSD assay. C5a was immobilized on a microplate with the neo-epitope anti-C5a antibody BNJ383. Refer to para 00223.

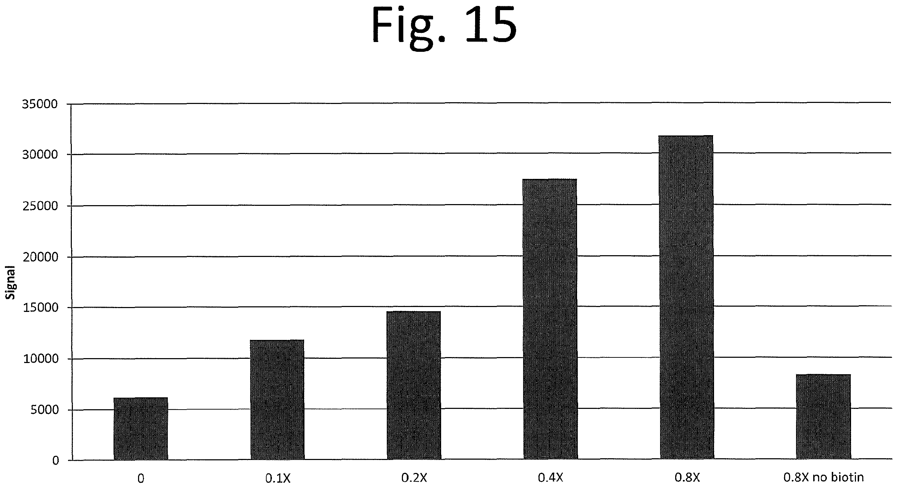

**Fig. 15** is a bar graph, which illustrates that the C5a generation decreases as bio-C3b was titrated from 0.8x, to 0.1x. Controls were the absence of bio-Cb3 and, Cb3 that had not been modified with biotin. These data demonstrate that C5a production (C5 convertase activity) is dependent on the amount of bio-C3b immobilized on the streptavidin-coated spheres.

### Example 11

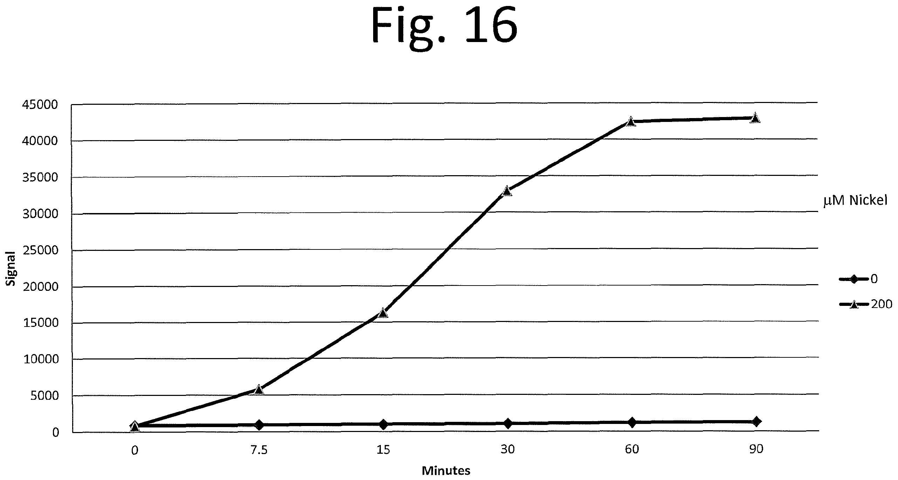

Example 11 demonstrates that the C5 convertase activity is dependent on the presence of Ni⁺². The assay was performed in a 96-well microplate. The results are shown in **Fig. 16****.** The Y-axis shows the ECL signal generated from a secondary anti-mouse goat antibody modified with SULFO-TAG™. The X-axis shows the time in minutes.

The ninety-six well microplate with C5 convertase immobilized on streptavidin-coated wells was prepared as in Example 2. There were 0.4 pmoles of bio-C3b per well; Factor B was 50 nM; C5 was 10 nM, Factor D was 10 nM, and NiCl₂ was 0 or 200 µM.

C5a detection was with an MSD assay. C5a was immobilized on a microplate with the neo-epitope anti-C5a antibody BNJ383. The amount of C5a immobilized on the plate was measured using an anti-C5a mouse antibody, and a secondary anti-mouse goat antibody modified with SULFO-TAG™. (para 00223 for details)

**Fig. 16** is a graph showing the results in the presence and the absence of NiCl₂. The Y-axis shows the ECL signal from the SULFO-TAG™ labeled secondary goat antibody. The X-axis shows the ratio of bio-C3b time in minutes. The results demonstrate that NiCl₂ is required for C5 convertase activity. In the presence of NiCl₂ the ECL signal increased with time, reaching a plateau of about 43,000 at about 60 minutes, whereas in the absence of NiCl₂ no ECL signal was observed.

### Other Embodiments

The foregoing description discloses only exemplary embodiments of the disclosure. It is to be understood that while several embodiments have been described in conjunction with the detailed description thereof, the foregoing description is intended to illustrate and not limit the scope of the appended claims. Other aspects, advantages, and modifications are within the scope of the appended claims. Thus, while only certain embodiments have been illustrated and described, many modifications and changes will occur to those skilled in the art.

## Claims

1. A method for measuring the protease activity of C3 convertase comprising the steps of:
a. covalently attaching biotin to C3b to produce biotinylated-C3b (bio-C3b);
b. binding biotinylated-C3b to a biotin binding protein immobilized on a solid phase;
c. incubating the immobilized biotinylated-C3b, Factor D, and Factor B in a buffer to form a C3 convertase;
d. transferring C3 to the buffer to cleave C3 with the convertase to form a C3a and C3b; and
e. measuring the amount of C3a with an immunoassay, wherein each of the individual components bio-C3b, Factor B, Factor D, and C3 are substantially homogeneous.

2. A method for measuring the protease activity of C5 convertase comprising the steps of:
a. covalently attaching biotin to C3b to produce biotinylated-C3b;
b. binding biotinylated-C3b to a biotin binding protein immobilized on a solid phase;
c. incubating the immobilized biotinylated-C3b, Factor D, and Factor B in a buffer to form C5 convertase;
d. transferring C5 to the buffer, and cleaving C5 with the convertase to form a C5a and C5b; and
e. measuring the amount of the C5a with an immunoassay,
wherein each of the individual components bio-C3b, Factor B, Factor D, and C5 are substantially homogeneous.

3. The method of claims 1 or 2, wherein the biotin-binding protein is selected from the group consisting of avidin, streptavidin and neutravidin, preferably streptavidin.

4. The method of claims 1 or 2, wherein the buffer further comprises properdin.

5. The method of claims 1 or 2, wherein the solid phase is a well in a microplate coated with a biotin binding protein, or wherein the solid phase comprises spheres coated with a biotin binding protein, optionally wherein the spheres are microspheres or nanospheres.

6. The method of claim 1, wherein the immunoassay comprises a step of detecting C3a with an antibody having a high affinity for C3a, and a low affinity for C3.

7. The method of claim 6, wherein the antibody is a neo-epitope antibody, or wherein the antibody has a Kd from about 10⁻⁶ to about 10⁻¹² for C3a, and a Kd from about 10⁻³ to about 10⁻⁵ for C3.

8. The method of claim 2, wherein the immunoassay comprises a step of detecting C5a with an antibody having a high affinity for C5a, and a low affinity for C5.

9. The method of claim 8, wherein the antibody is a neo-epitope antibody, or wherein the antibody has a Kd from about 10⁻⁶ to about 10⁻¹² for C5a, and a Kd from about 10⁻³ to about 10⁻⁵ for C5.

10. A kit for measuring the protease activity of C3 convertase of the alternative pathway using substantially homogeneous components of the alternative complement pathway, the kit comprising:
a. substantially homogeneous biotinylated C3b;
b. a solid phase coated with a biotin binding protein;
c. substantially homogeneous Factor B, Factor D and C3; and,
d. an anti-C3a antibody.

11. The kit of claim 10, further comprising properdin.

12. The kit of claim 10, wherein the solid phase is a well in a microplate coated with a biotin binding protein, or wherein the solid phase comprises spheres coated with a biotin binding protein, optionally wherein the spheres are microspheres or nanospheres.

13. A kit for measuring the protease activity of C5 convertase of the alternative pathway using substantially homogeneous components of the alternative complement pathway, the kit comprising:
a. substantially homogeneous biotinylated C3b;
b. a solid phase coated with a biotin binding protein;
c. substantially homogeneous Factor B, Factor D and C5; and,
d. an anti-C5a antibody.

14. The kit of claim 13, further comprising properdin.

15. The kit of claim 13, wherein the solid phase is a well in a microplate coated with a biotin binding protein, or wherein the solid phase comprises spheres coated with a biotin binding protein, optionally wherein the spheres are microspheres or nanospheres.

## Patentansprüche

1. Verfahren zum Messen der Protease-Aktivität von C3-Konvertase, umfassend die Schritte:
a. kovalentes Binden von Biotin an C3b unter Erhalt von biotinyliertes-C3b (bio-C3b);
b. Binden von biotinyliertes-C3b an ein Biotin bindendes Protein, das an einer Festphase immobilisiert ist;
c. Inkubieren des immobilisierten biotinyliertes-C3b, von Faktor D und Faktor B in einem Puffer unter Bildung einer C3-Konvertase;
d. Überführen von C3 in den Puffer zur Spaltung von C3 mit der Konvertase unter Erhalt eines C3a und C3b; und
e. Messen der Menge an C3a mit einem Immuntest,
wobei die einzelnen Komponenten bio-C3b, Faktor B, Faktor D und C3 weitgehend homogen sind.

2. Verfahren zum Messen der Protease-Aktivität von C5-Konvertase, umfassend die Schritte:
a. kovalentes Binden von Biotin an C3b unter Erhalt von biotinyliertes-C3ba;
b. Binden von biotinyliertes-C3b an ein Biotin bindendes Protein, das an einer Festphase immobilisiert ist;
c. Inkubieren des immobilisierten biotinyliertes-C3b, von Faktor D und Faktor B in einem Puffer unter Bildung von C5-Konvertase;
d. Überführen von C5 in den Puffer und Spalten von C5 mit der Konvertase unter Erhalt eines C5a und C5b; und
e. Messen der Menge des C5a mit einem Immuntest,
wobei die einzelnen Komponenten bio-C3b, Faktor B, Faktor D und C5 weitgehend homogen sind.

3. Verfahren nach Anspruch 1 oder 2, wobei das biotinbindende Protein aus der Gruppe bestehend aus Avidin, Streptavidin und Neutravidin, vorzugsweise Streptavidin ausgewählt ist.

4. Verfahren nach Anspruch 1 oder 2, wobei der Puffer ferner Properdin umfasst.

5. Verfahren nach Anspruch 1 oder 2, wobei es sich bei der Festphase um ein Well in einer Mikroplatte, die mit einem Biotin bindenden Protein beschichtet ist, handelt oder wobei die Festphase Kügelchen umfasst, die mit einem Biotin bindenden Protein beschichtet sind, gegebenenfalls wobei es sich bei den Kügelchen um Mikrokügelchen oder Nanokügelchen handelt.

6. Verfahren nach Anspruch 1, wobei der Immuntest einen Schritt umfasst, bei dem C3a mit einem Antikörper mit einer hohen Affinität für C3a und einer niedrigen Affinität für C3 nachgewiesen wird.

7. Verfahren nach Anspruch 6, wobei es sich bei dem Antikörper um einen Neoepitop-Antikörper handelt oder wobei der Antikörper eine Kd von etwa 10⁻⁶ bis etwa 10⁻¹² für C3a und eine Kd von etwa 10⁻³ bis etwa 10⁻⁵ für C3 aufweist.

8. Verfahren nach Anspruch 2, wobei der Immuntest einen Schritt umfasst, bei dem C5a mit einem Antikörper mit einer hohen Affinität für C5a und einer niedrigen Affinität für C5 nachgewiesen wird.

9. Verfahren nach Anspruch 8, wobei es sich bei dem Antikörper um einen Neoepitop-Antikörper handelt oder wobei der Antikörper eine Kd von etwa 10⁻⁶ bis etwa 10⁻¹² für C5a und eine Kd von etwa 10⁻³ bis etwa 10⁻⁵ für C5 aufweist.

10. Kit zum Messen der Protease-Aktivität von C3-Konvertase des alternativen Wegs unter Verwendung weitgehend homogener Komponenten des alternativen Komplementwegs, wobei das Kit Folgendes umfasst:
a. weitgehend homogenes biotinyliertes C3b;
b. eine Festphase, die mit einem Biotin bindenden Protein beschichtet ist;
c. weitgehend homogenen Faktor B, Faktor D bzw. weitgehend homogenes C3; und
d. einen Anti-C3a-Antikörper.

11. Kit nach Anspruch 10, ferner umfassend Properdin.

12. Kit nach Anspruch 10, wobei es sich bei der Festphase um ein Well in einer Mikroplatte, die mit einem Biotin bindenden Protein beschichtet ist, handelt oder wobei die Festphase Kügelchen umfasst, die mit einem Biotin bindenden Protein beschichtet sind, gegebenenfalls wobei es sich bei den Kügelchen um Mikrokügelchen oder Nanokügelchen handelt.

13. Kit zum Messen der Protease-Aktivität von C5-Konvertase des alternativen Wegs unter Verwendung weitgehend homogener Komponenten des alternativen Komplementwegs, wobei das Kit Folgendes umfasst:
a. weitgehend homogenes biotinyliertes C3b;
b. eine Festphase, die mit einem Biotin bindenden Protein beschichtet ist;
c. weitgehend homogenen Faktor B, Faktor D bzw. weitgehend homogenes C5; und
d. einen Anti-C5a-Antikörper.

14. Kit nach Anspruch 13, ferner umfassend Properdin.

15. Kit nach Anspruch 13, wobei es sich bei der Festphase um ein Well in einer Mikroplatte, die mit einem Biotin bindenden Protein beschichtet ist, handelt oder wobei die Festphase Kügelchen umfasst, die mit einem Biotin bindenden Protein beschichtet sind, gegebenenfalls wobei es sich bei den Kügelchen um Mikrokügelchen oder Nanokügelchen handelt.

## Revendications

1. Procédé de mesure de l'activité protéase d'une C3 convertase comprenant les étapes de :
a. liaison covalente de biotine à C3b pour produire C3b biotinylé (bio-C3b) ;
b. liaison de C3b biotinylé à une protéine de liaison de biotine immobilisée sur une phase solide ;
c. incubation de C3b biotinylé immobilisé, de facteur D, et de facteur B dans un tampon pour former une C3 convertase ;
d. transfert de C3 dans le tampon pour cliver C3 avec la convertase pour former C3a et C3b ; et
e. mesure de la quantité de C3a avec un immunodosage,
dans lequel chacun des composants individuels bio-C3b, facteur B, facteur D, et C3 est sensiblement homogène.

2. Procédé de mesure de l'activité protéase de C5 convertase comprenant les étapes de :
a. liaison covalente de biotine à C3b pour produire C3b biotinylé ;
b. liaison de C3b biotinylé à une protéine de liaison de biotine immobilisée sur une phase solide ;
c. incubation de C3b biotinylé immobilisé, de facteur D, et de facteur B dans un tampon pour former une C5 convertase ;
d. transfert de C5 dans le tampon et clivage de C5 avec la convertase pour former C5a et C5b ; et
e. mesure de la quantité de C5a avec un immunodosage,
dans lequel chacun des composants individuels bio-C3b, facteur B, facteur D, et C3 sont sensiblement homogènes.

3. Procédé selon les revendications 1 ou 2, dans lequel la protéine de liaison de biotine est choisie dans le groupe constitué de l'avidine, la streptavidine et la neutravidine, de préférence la streptavidine.

4. Procédé des revendications 1 ou 2, dans lequel le tampon comprend en outre de la properdine.

5. Procédé selon les revendications 1 ou 2, dans lequel la phase solide est un puits dans une microplaque revêtue avec une protéine de liaison de biotine, ou dans lequel la phase solide comprend des sphères enrobées avec une protéine de liaison de biotine, les sphères étant facultativement des microsphères ou des nanosphères.

6. Procédé selon la revendication 1, dans lequel l'immunodosage comprend une étape de détection de C3a avec un anticorps ayant une affinité élevée pour C3a, et une faible affinité pour C3.

7. Procédé selon la revendication 6, dans lequel l'anticorps est un anticorps contre un néo-épitope, ou dans lequel l'anticorps a un Kd d'environ 10⁻⁶ à environ 10⁻¹² pour C3a, et un Kd d'environ 10⁻³ à environ 10⁻⁵ pour C3.

8. Procédé selon la revendication 2, dans lequel l'immunodosage comprend une étape de détection de C5a avec un anticorps ayant une affinité élevée pour C5a, et une faible affinité pour C5.

9. Procédé selon la revendication 8, dans lequel l'anticorps est un anticorps contre un néo-épitope, ou dans lequel l'anticorps a un Kd d'environ 10⁻⁶ à environ 10⁻¹² pour C5a, et un Kd d'environ 10⁻³ à environ 10⁻⁵ pour C5.

10. Trousse pour mesurer l'activité protéase de C3 convertase de la voie alterne en utilisant des composants sensiblement homogènes de la voie alterne du complément, la trousse comprenant :
a. C3b biotinylé sensiblement homogène ;
b. une phase solide revêtue avec une protéine de liaison de biotine ;
c. du facteur B, du facteur D et C3 sensiblement homogènes ; et,
d. un anticorps anti-C3a.

11. Trousse selon la revendication 10, comprenant en outre de la properdine.

12. Trousse selon la revendication 10, dans laquelle la phase solide est un puits dans une microplaque revêtue avec une protéine de liaison de biotine, ou dans laquelle la phase solide comprend des sphères enrobées avec une protéine de liaison de biotine, les sphères étant facultativement des microsphères ou des nanosphères.

13. Trousse pour mesurer l'activité protéase de C5 convertase de la voie alterne en utilisant des composants sensiblement homogènes de la voie alterne du complément, la trousse comprenant :
a. C3b biotinylé sensiblement homogène ;
b. une phase solide revêtue avec une protéine de liaison de biotine ;
c. du facteur B, du facteur D et C5 sensiblement homogènes ; et,
d. un anticorps anti-C5a.

14. Trousse selon la revendication 13, comprenant en outre de la properdine.

15. Trousse selon la revendication 13, dans laquelle la phase solide est un puits dans une microplaque revêtue avec une protéine de liaison de biotine, ou dans lequel la phase solide comprend des sphères enrobées avec une protéine de liaison de biotine, les sphères étant facultativement des microsphères ou des nanosphères.
